(19) **Europäisches Patentamt / European Patent Office / Office européen des brevets**

(11) **EP 1 463 490 B1**

(12) **EUROPEAN PATENT SPECIFICATION**

(45) Date of publication and mention
of the grant of the patent:
**14.12.2011 Bulletin 2011/50**

(51) Int Cl.:
*A61K 9/70* (2006.01)    *A61K 9/00* (2006.01)
*A61K 47/36* (2006.01)

(21) Application number: **02769061.9**

(22) Date of filing: **11.10.2002**

(86) International application number:
**PCT/US2002/032542**

(87) International publication number:
**WO 2003/030881 (17.04.2003 Gazette 2003/16)**

(54) **GLUCAN BASED FILM DELIVERY SYSTEMS**

AUF GLUCAN-FILM BASIERTE WIRKSTOFFVERABREICHUNGSSYSTEME

SYSTEMES DE DISTRIBUTION DE SUBSTANCE ACTIVE SOUS FORME DE FILM A BASE DE GLUCANE

(84) Designated Contracting States:
**AT BE BG CH CY CZ DE DK EE ES FI FR GB GR IE IT LI LU MC NL PT SE SK TR**

(30) Priority: **12.10.2001 US 328868 P**
**14.02.2002 US 74272**
**11.04.2002 US 371940 P**
**07.06.2002 US 386937 P**

(43) Date of publication of application:
**06.10.2004 Bulletin 2004/41**

(73) Proprietor: **MonoSolRX, LLC**
**Portage IN 46368 (US)**

(72) Inventors:
• **Yang, Robert K.**
**Flushing, NY 11355 (US)**
• **Fuisz, Richard C.**
**McLean, VA 22101 (US)**

(74) Representative: **Raggers, René John**
**Exter Polak & Charlouis B.V.**
**P.O. Box 3241**
**2280 GE Rijswijk (NL)**

(56) References cited:
WO-A-00/18365    WO-A-01/70194
US-A- 4 562 020    US-A- 4 623 394
US-A- 4 631 837    US-A- 4 927 636
US-A- 4 981 693    US-A- 5 411 945
US-A- 5 518 902

• LAZARIDOU A ET AL: "Thermophysical properties of chitosan, chitosan-starch and chitosan-pullulan films near the glass transition" CARBOHYDRATE POLYMERS, APPLIED SCIENCE PUBLISHERS, LTD. BARKING, GB, vol. 48, no. 2, 1 May 2002 (2002-05-01), pages 179-190, XP004333230 ISSN: 0144-8617

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

**FIELD OF THE INVENTION**

The invention relates to film delivery systems, especially suitable for oral delivery, which can be formed during manufacture in the form of large film strips or sheets and subsequently cut into uniform dosage units, each dosage unit being uniform in content and having distributed therein a glucan, such as pullulan, a water-soluble polymer and optionally an active component.

**BACKGROUND OF RELATED TECHNOLOGY**

Self-supporting glucan films, such as those made from pullulan and elsinan, are known as being useful for the delivery of edible or ingestible components, for packaging and wrapping as well as other uses. For example, U.S. Patent No. 4,562,020 to Haijiya, et al. discloses a continuous process for producing such self-supporting glucan films formed from aqueous glucan solutions whereby the solution is deposited on a corona-treated plastic conveyor belt and air dried to form thin films. A variety of additional ingredients, such as flavors, colors, seasonings, cyclodextrine, protein, fat, vitamin, hormones, anti-genic substances, antibiotics, biologically active substances, viruses, lecithin, plasticizers, micro-organisms, spores, seeds and the like are disclosed as being useful additives.

Pullulan is a desirable material for forming edible films because of its high water solubility, rapid dissolution and excellent mouth-feel. However, due to its relatively low molecular weight as compared to many polymeric film forming materials, it is difficult to achieve films having low pullulan content. This is because, due to the low viscosity of pullulan, high amounts of pullulan must be present in the aqueous solution for good film casting. Generally, the pullulan content must be greater than 20% by weight in order to form a useful film. When insoluble additives are to be added to such pullulan solutions, even more pullulan must be used in order to achieve sufficient viscosity to prevent separation of the insoluble particulate from the solution prior to or during the film forming process. For example, many useful pharmaceutical and cosmetic active agents are water insoluble particulates. These particulates must be uniformly dispersed throughout the pullulan solution and must remain uniformly dispersed in the film forming process, such that the resultant films have uniform drug content. For example, by uniform content it is meant that unit dosages cut from larger pieces of film will not substantially vary in the amount of drug content. In particular, the drug content of any one unit dosage should not vary more than 10% in order to be acceptable for sale under regulations by the U.S. Federal Drug Administration (FDA) or other world regulatory authorities regulations.

Films made from pullulan to-date also suffer the limitation that in order to put higher drug levels in the film, increased pullulan content is necessary in order to achieve uniformity in film content.

U.S. Patent No. 4,927,636 to Haijiya, et al. discloses pullulan films which have decreased solubility in water. These films are made from a combination of pullulan and polyethylene glycol (PEG) which form an "association complex" to produce this effect. Polyethylene glycols within the molecular range of 400 to 10,000 are disclosed as useful. The ratio of pullulan to PEG is disclosed as being 1 part by weight (pbw) pullulan to 0.01 to 100 part by weight (pbw) PEG. This patent discloses that pullulan in combination with other water-soluble polymers does not form such an association complex useful for decreasing solubility and reducing adhesive and stickiness properties of aqueous pullulan.

U.S. Patent No. 5,411,945 to Ozaki, et al. discloses a pullulan binder composition made from a combination of pullulan and a mono-saccharide or lower molecular weight oligo-saccharide in a ratio of 85:15 to 65:35 pullulan/saccharide. These films are disclosed as being gradually dissolvable.

U.S. Patent No. 5,518,902 to Ozaki, et al. discloses high pullulan content products made by cultivating micro-organisms capable of producing pullulan at a pH exceeding 2.0 but not higher than 4.0 in a nutrient culture medium containing 10-20 w/v % of a polysaccharide to produce pullulan, while controlling the viscosity of the nutrient culture to below 30 cps.

U.S. Patent Application Publication No. 2001/0022964 A1 to Leung, et al. discloses edible films made from pullulan and which include anti-microbial effective amounts of essential oils. A variety of polymers may be used as film formers in addition to pullulan.

PCT Application No. WO 01/70194 discloses physiologically acceptable films that include a taste-masking agent, and a water-soluble polymer. The water-soluble polymer is suitably pullulan and optionally various other different film-forming polymers Cellulosic polymers are included in a lengthy listing of suitable materials. In the examples in WO 01/70194 various gums are used as additional film-forming material, in addition to pullulan. There is no specific disclosure OI suggestion in WO 01/70194 to select a glucan and a water-soluble cellulosic polymer for making an ingestible film composition.

Various drugs may be included in the films. The content of pullulan used is very high and the drug loading is very low, as is typical of pullulan films.

It would be desirable to have film products made from pullulan which can include high content active ingredients, such as pharmaceutical and/or cosmetic agents, and which have relatively low pullulan content. It would also be advantageous to provide water-soluble, edible films which have uniformity of active content, both in the manufacturing scale and in the unit dosage, final product form.

## SUMMARY OF THE INVENTION

The present invention seeks to attain low content, high pharmaceutical and/or cosmetic active content films which have enhanced flexibility, structural integrity and uniformity. The present invention also provides for a unique method of producing the inventive compositions such that uniform distribution of the compositional components are evenly distributed throughout the film. This process is described in detail in co-pending U.S. Patent Application No. 10/074,272, entitled "Thin Film with Non-Self Aggregating Uniform Heterogeneity and Drug Delivery Systems Made Therefrom".

In one aspect of the present invention there is included an ingestible water-soluble delivery system in the form of a film composition comprising a glucan, a water-soluble polymer comprising a cellulosic material, wherein said cellulosic material is selected from the group consisting of carboxymethyl cellulose, hydroxy methyl cellulose, hydroxyethyl cellulose, Hydroxypropyl cellulose hydroxypropylmethyl cellulose and combinations thereof, and an active component, wherein the ratio of glucan to water soluble polymer by weight is 10:1 1 to 1:5, and wherein the viscosity of the film composition is 400 cps to 100,000 cps, said viscosity preventing components from settling, which would adversely affect the uniformity of the film.

Desirably, a pharmaceutical and/or cosmetic active component is present in the delivery system in amounts effective for its intended use, desirably, due to the advantages of the aforementioned glucan/water-soluble polymer ratio, high amounts of active components may be present without disrupting the uniformity of content of the overall film and subsequently he individual dosage forms cut therefrom Significantly lower amounts of pullulan, as compared to conventional pullulan films, can be used while maintaining a high load of active components. Increased flexibility as compared to pullulan or the water-soluble polymers being used along is achieved, as well as enhanced tensile strength and overall structural integrity. Elongation of the resultant film is substantially unaffected as compared to pullulan alone.

In another aspect of the present invention there is included a pharmaceutical composition in the form of a film for enteral or topical administration, comprising a composition having a uniformly distributed combination of a glucan, a water-soluble polymer comprising a cellulosic material, wherein said cellulosic material is selected from the group consisting of carboxymethyl cellulose, hydroxyl methyl cellulose, hydroxyethyl cellulose, hydroxypropyl cellulose hydroxypropylmethyl cellulose and combinations thereof, a polar solvent, and a pharmaceutical active, wherein the ratio of glucan to water-soluble polymer by weight is 10:1 1 to 1:5, and wherein the viscosity of the film composition is 400 cps to 100,000 cps, said viscosity preventing components from settling, which would adversely affect the uniformity of the film, said composition in its dried film form maintaining the uniform distribution of components through the application of controlled bottom drying of the film.

In another aspect of the present invention there is included a pharmaceutical and/or cosmetic dosage form comprising a film having a uniformly dispersed composition comprising a glucan and a water-soluble polymer comprising a cellulosic material, wherein said cellulosic material is selected from the group consisting of carboxymethyl cellulose, hydroxyl methyl cellulose, hydroxyethyl cellulose, hydroxypropyl cellulose hydroxypropylmethyl cellulose and combinations thereof, a pharmaceutical and/or cosmetic active and a solvent, wherein the ratio of' glucan to water-soluble polymer by weight is 10:1 1 to 1:5, and wherein the viscosity of the film composition is 400 cps to 100,000 cps, said viscosity preventing components from settling, which would adversely affect the uniformity of the film, said film being formed by depositing a wet film of said composition onto a substrate surface and controllably drying the wet film from the side contacting the substrate to prevent self-aggregation and achieve compositional uniformity

In a further aspect of the present invention there is provided a pharmaceutical and/or cosmetic dosage form comprising an ingestible water-soluble film composition comprising a glucan and a water-soluble polymer, comprising a cellulosic material, wherein said cellulosic material is selected from the group consisting of carboxymethyl cellulose, hydroxyl methyl cellulose, hydroxyethyl cellulose, hydroxypropyl cellulose hydroxyprop,ylmethyl cellulose and combinations thereof, wherein the ratio of glucan to water-soluble polymer by weight is 10:1 1 to 1:5, and wherein the viscosity of the film composition is 400 cps to 100,000 cps, said viscosity preventing components from settling, which would adversely affect the uniformity of the film, said film being formed by depositing a wet film of said composition onto a substrate surface and controllably drying the wet film from the side contacting the substrate to prevent self-aggregation and achieve compositional uniformity to provide a film having no more than a 10% variance of a pharmaceutical and/or cosmetic active per unit area.

## BRIEF DESCRIPTION OF THE DRAWINGS

Figure 1 shows a side view of' a package containing a unit dosage film of the present invention.

Figure 2 shows a top view of two adjacently coupled packages containing individual unit dosage forms of the present invention, separated by a tearable perforation.

Figure 3 shows a side view of the adjacently coupled packages of Figure 2 arranged in a stacked configuration.

Figure 4 shows a perspective view of a dispenser for dispensing the packaged unit dosage forms, dispenser containing the packaged unit dosage forms in a stacked configuration.

Figure 5 is a schematic view of a roll of coupled unit dose packages of the present invention.

Figure 6 is a schematic view of an apparatus suitable for preparation of a pre-mix, addition of an active, and subsequent formation of the film.

Figure 7 is a schematic view of an apparatus suitable for drying the films of the present invention.

## DETAILED DESCRIPTION OF THE INVENTION

Glucans useful in the present invention include pullulan and elsinan. These materials substantially contain repeating maltotriose units and are produced by culturing a strain of species *Aureobasidium pullulans* or genus *Elsino* on a nutrient medium containing sugars under aeration and agitation conditions. The cellular debris is removed and the resultant supernatant is purified and filtrated to yield the resultant glucan. The molecular weight of the glucan may vary widely, but generally are commercially available at molecular weights of 50,000 to 100,000. Due to the relatively low molecular weight, water solubility is very high and attaining useful viscosities for film forming requires a high content of pullulan;

Water-soluble polymers useful in the present invention cellulosic materials, gums, proteins, starches, and combinations thereof.

Examples of cellulosic materials include, without limitation, carboxymethyl cellulose, hydroxyl methyl cellulose, hydroxyethyl cellulose, hydroxypropyl cellulose hydroxypropylmethyl cellulose, and combinations thereof.

Examples of water-soluble gums include gum arabic, xanthan gum, tragacanth, acacia, carageenan, guar gum, locust bean gum, pectin, alginates and combinations thereof.

Examples of other polymeric materials include polyvinyl alcohol, polyacrylic acid, polyvinyl pyrrolidone, poly (meth)acrylate, poly(meth)copolymers and combinations thereof.

Useful starches include gelatinized, modified or unmodified starches. The source of the starches may vary and include tapioca, rice, corn, potato, wheat and combinations thereof.

Useful water-soluble protein polymers gelatin, zein, gluten, soy protein, soy protein isolate, whey protein, whey protein isolate, casein, levin, collagen and combinations thereof Additional water-soluble polymers include dextrin, dextran and combinations thereof, as well as chitin, chitosin or combinations thereof, and polydextrose.

To achieve the desired results of the present invention of film content uniformity, the ratio of glucan to water-soluble polymer is 10:1 to 1:5.

The ingestible water-soluble delivery system of'the present invention further include an active component selected from cosmetic agents, pharmaceutical agents, bioactive agents and combinations thereof The active component may be present in any amount effective for the intended treatment. It is particularly desirable and an advantage of the present invention that the active component can be included in high loads. For example, the active component may be present in amounts up to 60% by weight of the total composition and desirably in amounts of 0.01% to 50% by weight of total composition.

The edible water-soluble delivery system of the present invention further includes one or more members selected from taste-masking agents, plasticizing agents, surfactants, emulsifying agents, thickening agents, binding agents, cooling agents, saliva stimulating agents, sweetening agents, antimicrobial agents and combinations thereof.

## Uses of Thin Films

The thin films of the present invention are well suited for many uses. The high degree of uniformity of the components of the film makes them particularly well suited for incorporating pharmaceuticals. Furthermore, the polymers used in construction of the films may be chosen to allow for a range of disintegration times for the films. A variation or extension in the time over which a film will disintegrate may achieve control over the rate that the active is released, which may allow for a sustained release delivery system. In addition, the films may be used for the administration of an active to any of several body surfaces, especially those including mucous membranes, such as oral, anal, vaginal, ophthalmological, the surface of a wound, either on a skin surface or within a body such as during surgery, and similar surfaces.

The films may be used to orally administer an active. This is accomplished by preparing the films as described above and introducing them to the oral cavity of a mammal. This film may be prepared and adhered to a second or support layer from which it is removed prior to use, i.e. introduction to the oral cavity. An adhesive may be used to attach the film to the support or backing material which may be any of those known in the art, and is preferably not water soluble. If an adhesive is used, it will desirably be a food grade adhesive that is ingestible and does not alter the properties of the active. Mucoadhesive compositions are particularly useful. The film compositions in many cases serve as mucoadhesives themselves.

The films may be applied under or to the tongue of the mammal. When this is desired, a specific film shape, corresponding to the shape of the tongue may be preferred. Therefore the film may be cut to a shape where the side of the film corresponding to the back of the tongue will be longer than the side corresponding to the front of the tongue. Specifically, the desired shape may be that of a triangle or trapezoid. Desirably, the film will adhere to the oral cavity preventing it from being ejected from the oral cavity and permitting more of the active to be introduced to the oral cavity as the film dissolves.

Another use for the films of the present invention takes advantage of the films' tendency to dissolve quickly when introduce to a liquid. An active may be introduced to a liquid by preparing a film in accordance with the present invention, introducing it to a liquid, and allowing it to dissolve. This may be used either to prepare a liquid dosage form of an active, or to flavor a beverage.

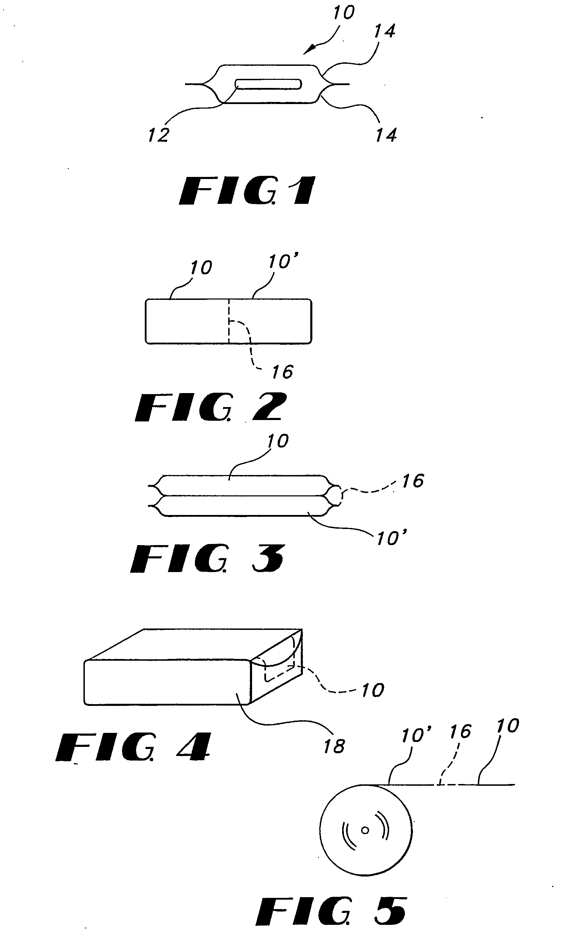

The films of the present invention are desirably packaged in sealed, air and moisture resistant packages to protect the active from exposure oxidation, hydrolysis, volatilization and interaction with the environment. Referring to Figure 1, a packaged pharmaceutical dosage unit 10, includes each film 12 individually wrapped in a pouch or between foil and/or plastic laminate sheets 14. As depicted in Figure 2, the pouches 10, 10' can be linked together with tearable or perforated joints 16. The pouches 10, 10' may be packaged in a roll as depicted in Figure 5 or stacked as shown in Figure 3 and sold in a dispenser 18 as shown in Figure 4. The dispenser may contain a full supply of the medication typically prescribed for the intended therapy, but due to the thinness of the film and package, is smaller and more convenient than traditional bottles used for tablets, capsules and liquids. Moreover, the films of the present invention dissolve instantly upon contact with saliva or mucosal membrane areas, eliminating the need to wash the dose down with water.

Desirably, a series of such unit doses are packaged together in accordance with the prescribed regimen or treatment, e.g., a 10-90 day supply, depending on the particular therapy. The individual films can be packaged on a backing and peeled off for use.

## Rheology and Films Properties

For the purposes of the present invention the term non-self-aggregating uniform heterogeneity refers to the ability of the films of the present invention, which are formed from one or more components in addition to a polar solvent, to provide a substantially reduced occurrence of, i.e. little or no, aggregation or conglomeration of components within the film as is normally experienced when films are formed by conventional drying methods such as a high-temperature air-bath using a drying oven, drying tunnel, vacuum drier, or other such drying equipment. The term heterogeneity, as used in the present invention, includes films that will incorporate a single component, such as a polymer, as well as combinations of components, such as a polymer and an active. Uniform heterogeneity includes the substantial absence of aggregates or conglomerates as is common in conventional mixing and heat drying methods used to form films.

Furthermore, the films of the present invention have a substantially uniform thickness, which is also not provided by the use of conventional drying methods used for drying water-based polymer systems. The absence of a uniform thickness detrimentally affects uniformity of component distribution throughout the area of a given film.

The film products of the present invention are produced by a combination of a properly selected polymer and a polar solvent, optionally including an active ingredient as well as other fillers known in the art. These films provide a non-self-aggregating uniform heterogeneity of the components within them by utilizing a selected casting or deposition method and a controlled drying process. Examples of controlled drying processes include, but are not limited to, the use of the apparatus disclosed in U.S. Patent No. 4,631,837 to Magoon ("Magoon"), as well as hot air impingement across the bottom substrate and bottom heating plates. Another drying technique for obtaining the films of the present invention is controlled radiation drying, in the absence of uncontrolled air currents, such as infrared and radio frequency radiation (i.,e. microwaves).

The objective of the drying process is to provide a method of drying the films that avoids complications, such as the noted "rippling" effect, that are associated with conventional drying methods and which initially dry the upper surface of the film, trapping moisture inside. In conventional oven drying methods, as the moisture trapped inside subsequently evaporates, the top surface is altered by being ripped open and then reformed These complications are avoided by the present invention, and a uniform film is provided by drying the bottom surface of the film first or otherwise preventing the formation of polymer film formation (skin) on the top surface of the film prior to drying the depth of the film. This may be achieved by applying heat to the bottom surface of the film with substantially no top air flow, or alternatively by the introduction of controlled microwaves to evaporate the water or other polar solvent within the film, again with substantially no top air flow. Yet alternatively, drying may be achieved by using balanced fluid flow, such as balanced air flow, where the bottom and top air flows are controlled to provide a uniform film. In such a case, the air flow directed at the top of the film should not create a condition which would cause movement of particles present in the wet film, due to forces generated by the air currents. Additionally, air currents directed at the bottom of the film should desirably be controlled such that the film does not lift up due to forces from the air. Uncontrolled air currents, either above or below the film, can create non-uniformity in the final film products, The humidity level of the area surrounding the top surface may also be appropriately adjusted to prevent premature closure or skinning of the polymer surface,

This manner of drying the films provides several advantages. Among these are the faster drying times and a more uniform surface of the film, as well as uniform distribution of components for any given area in the film. In addition, the faster drying time allows viscosity to quickly build within the film, further encouraging a uniform distribution of components and decrease in aggregation of components in the final film product. Desirably, the drying of the film will occur within ten minutes or fewer, or more desirably within five minutes or fewer.

The present invention yields exceptionally uniform film products when attention is paid to reducing the aggregation of the compositional components. By avoiding the introduction of and eliminating excessive air in the mixing process, selecting polymers and solvents to provide a controllable viscosity and by drying the film in a rapid manner from the bottom up, such films result.

The products and processes of the present invention rely on the interaction among various steps of the production of the films in order to provide films that substantially reduce the self-aggregation of the components within the films. Specifically, these steps include the particular method used to form the film, making the composition mixture to prevent air bubble inclusions, controlling the viscosity of the film forming composition and the method of drying the film. More particularly, a greater viscosity of components in the mixture is particularly useful when the active is not soluble in the selected polar solvent in order to prevent the active from settling out. However, the viscosity must not be too great as to hinder or prevent the chosen method of casting, which desirably includes reverse roll coating due to its ability to provide a film of substantially consistent thickness.

In addition to the viscosity of the film or film-forming components or matrix, there are other considerations taken into account by the present invention for achieving desirable film uniformity. For example, stable suspensions are achieved which prevent solid (such as drug particles) sedimentation in non-colloidal applications. One approach provided by the present invention is to balance the density of the particulate ($\rho_p$) and the liquid phase ($\rho_1$) and increase the viscosity of the liquid phase ($\mu$). For an isolated particle, Stokes law relates the terminal settling velocity (Vo) of a rigid spherical body of radius (r) in a viscous fluid, as follows:

$$V_o = (2gr^r)(\rho_p - \rho_l)/9\mu$$

At high particle concentrations, however, the local particle concentration will affect the local viscosity and density. The viscosity of the suspension is a strong function of solids volume fraction, and particle-particle and particle-liquid interactions will further hinder settling velocity.

Stokian analyses has shown that the incorporation of a third phase, dispersed air or nitrogen, for example, promotes suspension stability. Further, increasing the number of particles leads to a hindered settling effect based on the solids volume fraction. In dilute particle suspensions, the rate of sedimentation, v, can be expressed as:

$$v/V_o = 1/(1 + \kappa\varphi)$$

where $\kappa$ = a constant, and $\varphi$ is the volume fraction of the dispersed phase. More particles suspended in the liquid phase results in decreased velocity. Particle geometry is also an important factor since the particle dimensions will affect particle-particle flow interactions.

Similarly, the viscosity of the suspension is dependent on the volume fraction of dispersed solids. For dilute suspensions of non-interaction spherical particles, an expression for the suspension viscosity can be expressed as:

$$\mu/\mu_o = 1 + 2.5\phi$$

where $\mu_o$ is the viscosity of the continuous phase and $\phi$ is the solids volume fraction. At higher volume fractions, the viscosity of the dispersion can be expressed as

$$\mu/\mu_o = 1 + 2.5\varphi + C_1\varphi^2 + C_2\varphi^3 + \ldots..$$

where C is a constant.

The viscosity of the liquid phase is critical and is desirably modified by customizing the liquid composition to a viscoelastic non-Newtonian fluid with low yield stress values. This is the equivalent of producing a high viscosity continuous phase at rest. Formation of a viscoelastic or a highly structured fluid phase provides additional resistive forces to particle sedimentation. Further, flocculation or aggregation can be controlled minimizing particle-particle interactions. The net effect would be the preservation of a homogeneous dispersed phase.

The addition of hydrocolloids to the aqueous phase of the suspension increases viscosity, may produce viscoelasticity and can impart stability depending on the type of hydrocolloid, its concentration and the particle composition, geometry, size, and volume fraction. The particle size distribution of the dispersed phase needs to be controlled by selecting the smallest realistic particle size in the high viscosity medium, i.e., <500$\mu$m. The presence of a slight yield stress or elastic body at low shear rates may also induce permanent stability regardless of the apparent viscosity. The critical particle diameter can be calculated from the yield stress values. In the case of isolated spherical particles, the maximum shear stress developed in settling through a medium of given viscosity can be given as

$$\tau_{max} = 3V\mu/2r$$

For pseudoplastic fluids, the viscosity in this shear stress regime may well be the zero shear rate viscosity at the Newtonian plateau.

A stable suspension is an important characteristic for the manufacture of a pre-mix composition which is to be fed into the film casting machinery film, as well as the maintenance of this stability in the wet film stage until sufficient drying has occurred to lock-in the particles and matrix into a sufficiently solid form such that uniformity is maintained. For viscoelastic fluid systems, a rheology that yields stable suspensions for extended time period, such as 24 hours, must be balanced with the requirements of highspeed film casting operations. A desirable property for the films is shear thinning or pseudoplasticity, whereby the viscosity decreases with increasing shear rate. Time dependent shear effects such as thixotropy are also advantageous. Structural recovery and shear thinning behavior are important properties, as is the ability for the film to self-level as it is formed.

The rheology requirements for the inventive compositions and films are quite severe. This is due to the need to produce a stable suspension of particles, for example 30-60 wt%, in a viscoelastic fluid matrix with acceptable viscosity values throughout a broad shear rate range. During mixing, pumping, and film casting, shear rates in the range of 10-10$^5$ sec.$^{-1}$ may be experienced and pseudoplasticity is the preferred embodiment.

In film casting or coating, rheology is also a defining factor with respect to the ability to form films with the desired uniformity. Shear viscosity, extensional viscosity, viscoelasticity, structural recovery will influence the quality of the film. As an illustrative example, the leveling of shear-thinning pseudoplastic fluids has been derived as

$$\alpha^{(n-1/n)} = \alpha_o^{(n-1/n)} - ((n-1)/(2n-1))(\tau/K)^{1/n} (2\pi/\lambda)^{(3+n)/n} h^{(2n+1)/n} t$$

where $\alpha$ is the surface wave amplitude, $\alpha_o$ is the initial amplitude, $\lambda$ is the wavelength of the surface roughness, and both "n" and "K" are viscosity power law indices. In this example, leveling behavior is related to viscosity, increasing as n decreases, and decreasing with increasing K.

Desirably, the films or film-forming compositions of the present invention have a very rapid structural recovery, i.e. as the film is formed during processing, it doesn't fall apart or become discontinuous in its structure and compositional uniformity. Such very rapid structural recovery retards particle settling and sedimentation. Moreover, the films or film-forming compositions of the present invention are desirably shear-thinning pseudoplastic fluids. Such fluids with consideration of properties, such as viscosity and elasticity, promote thin film formation and uniformity,

Thus, uniformity in the mixture of components depends upon numerous variables. As described herein, viscosity of the components, the mixing techniques and the rheological properties of the resultant mixed composition and wet casted film are important aspects of the present invention. Additionally, control of particle size and particle shape are further considerations. Desirably, the size of the particulate a particle size of 150 microns or less, for example 100 microns or less. Moreover, such particles maybe spherical, substantially spherical, or non-spherical, such as irregularly shaped particles or ellipsoidally shaped particles. Ellipsoidally shaped particles or ellipsoids are desirable because of their ability to maintain uniformity in the film forming matrix as they tend to settle to a lesser degree as compared to spherical particles

Although a variety of different polymers may be used, it is desired to select polymers to provide a desired viscosity of the mixture prior to drying. For example, if the active or other components are not soluble in the selected solvent, a polymer that will provide a greater viscosity is desired to assist in maintaining uniformity On the other hand, if the components are soluble in the solvent, a polymer that provides a lower viscosity may be preferred.

The polymer plays an important role in affecting the viscosity of the film. Viscosity is one property of a liquid that controls the stability of the active in an emulsion, a colloid or a suspension. The viscosity of the matrix will vary from 400 cps ("cps" or "centipoise") to 100,000 cps, preferably from 800 cps to 60,000 cps, and most preferably from 1,000 cps to 40,000 cps. Desirably, the viscosity of the film-foxming matrix will rapidly increase upon initiation of the drying process.

The viscosity may be adjusted based on the selected active depending on the other components within the matrix. For example, if the component is not soluble within the selected solvent, a proper viscosity may be selected to prevent the component from settling which would adversely affect the uniformity of the resulting film. The viscosity may be adjusted in different ways. To increase viscosity of the film matrix, the polymer may be chosen of a higher molecular weight or crosslinkers may be added, such as salts of calcium, sodium and potassium. The viscosity may also be adjusted by adjusting the temperature or by adding a viscosity increasing component. Components that will increase the viscosity or stabilize the emulsion/suspension include higher molecular weight polymers and polysaccharides and gums, which include without limitation, alginate, carrageenan, hydroxypropyl methyl cellulose, locust bean gum, guar gum, xanthan gum, dextran, gum arabic, gellan gum and combinations thereof.

### Film Component Mixing:

A number of techniques may be employed in the mixing stage to prevent bubble inclusions in the final film. To provide a composition mixture with substantially no air bubble formation in the final product, anti-foaming or surface-tension reducing agents are employed. Additionally, the speed of the mixture is desirably controlled to prevent cavitation of the mixture in a manner which pulls air into the mix. Finally, air bubble reduction can further be achieved by allowing the mix to stand for a sufficient time for bubbles to escape prior to drying the film. Desirably, the inventive process first forms a masterbatch of film-forming components without active ingredients such as drug particles or volatile materials such as flavor oils. The actives are added to smaller mixes of the masterbatch just prior to casting. Thus, the masterbatch pre-mix can be allowed to stand for a longer time without concern for instability in drug or other ingredients.

When the matrix is formed including the film-forming polymer and polar solvent in addition to any additives and the active ingredient, this may be done in a number of steps. For example, the ingredients may all be added together or a pre-mix may be prepared. The advantage of a pre-mix is that all ingredients except for the active may be combined in advance, with the active added just prior to formation of the film. This is especially important for actives that may degrade with prolonged exposure to water, air or another polar solvent.

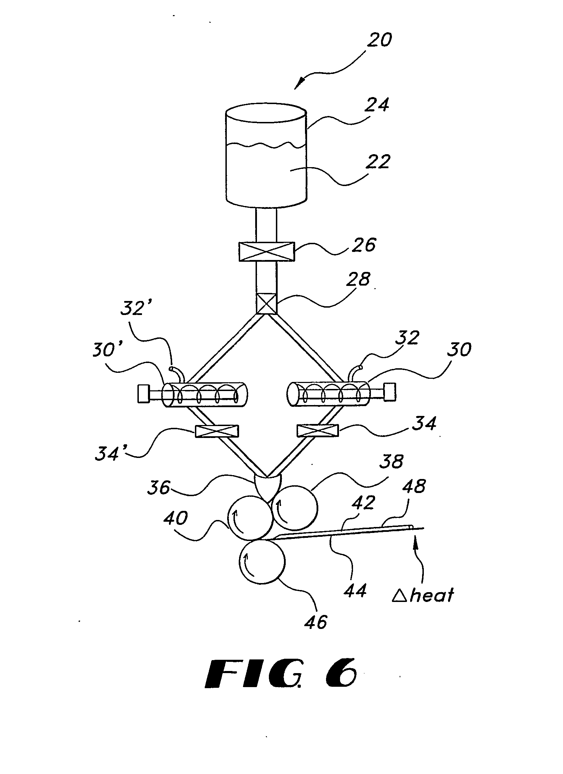

Figure 6 shows an apparatus 20 suitable for the preparation of a pre-mix, addition of an active and subsequent formation of a film. The pre-mix or master batch 22, which includes the film-forming polymer, polar solvent, and any other additives except a drug active is added to the master batch feed tank 24. The components for pre-mix or master batch 22 are desirably formed in a mixer (not shown) prior to their addition into the master batch feed tank 24. Then a pre-determined amount of the master batch is controllably fed via a first metering pump 26 and control valve 28 to either or both of the first and second mixers, 30, 30'. The present invention, however, is not limited to the use of two mixers, 30, 30', and any number of mixers may suitably be used. Moreover, the present invention is not limited to any particular sequencing of the mixers 30, 30', such as parallel sequencing as depicted in Figure 6, and other sequencing or arrangements of mixers, such as series or combination of parallel and series, may suitably be used. The required amount of the drug or other ingredient, such as a flavor, is added to the desired mixer through an opening, 32, 32', in each of the mixers, 30, 30'. Desirably, the residence time of the pre-mix or master batch 22 is minimized in the mixers 30, 30'. While complete dispersion of the drug into the pre-mix or master batch 22 is desirable, excessive residence times may result in leaching or dissolving of the drug, especially in the case for a soluble drug. Thus, the mixers 30, 30' are often smaller, i.e. lower residence times, as compared to the primary mixers (not shown) used in forming the pre-mix or master batch 22. After the drug has been blended with the master batch pre-mix for a sufficient time to provide a uniform matrix, a specific amount of the uniform matrix is then fed to the pan 36 through the second metering pumps, 34, 34'. The metering roller 38 determines the thickness of the film 42 and applies it to the application roller. The film 42 is finally formed on the substrate 44 and carried away via the support roller 46.

**Forming the Film**

The films of the present invention must be formed into a sheet prior to drying. After the desired components are combined to form a multi-component matrix, including the polymer, water, and an active or other components as desired, the combination is formed into a sheet or film, by any method known in the art such as extrusion, coating, spreading, casting or drawing the multi-component matrix. If a multi-layered film is desired, this may be accomplished by co-extruding more than one combination of components which may be of the same or different composition. A multi-layered film may also be achieved by coating, spreading, or casting a combination onto an already formed film layer.

Although a variety of different film-forming techniques may be used, it is desirable to select a method that will provide a flexible film, such as reverse roll coating. The flexibility of the film allows for the sheets of film to be rolled and transported for storage or prior to being cut into individual dosage forms. Desirably, the films will also be self-supporting or in other words able to maintain their integrity and structure in the absence of a separate support. Furthermore, the films of the present invention may be selected of materials that are edible or ingestible.

Coating or casting methods are particularly useful for the purpose of forming the films of the present invention. Specific examples include reverse roll coating, gravure coating, immersion or dip coating, metering rod or meyer bar coating, slot die or extrusion coating, gap or knife over roll coating, air knife coating, curtain coating, or combinations thereof, especially when a multi-layered film is desired.

Roll coating, or more specifically reverse roll coating, is particularly desired when forming films in accordance with the present invention. This procedure provides excellent control and uniformity of the resulting films, which is desired in the present invention. In this procedure, the coating material is measured onto the applicator roller by the precision setting of the gap between the upper metering roller and the application roller below it. The coating is transferred from the application roller to the substrate as it passes around the support roller adjacent to the application roller. Both three roll and four roll processes are common.

The gravure coating process relies on an engraved roller running in a coating bath, which fills the engraved dots or lines of the roller with the coating material. The excess coating on the roller is wiped off by a doctor blade and the coating is then deposited onto the substrate as it passes between the engraved roller and a pressure roller.

Offset Gravure is common, where the coating is deposited on an intermediate roller before transfer to the substrate.

In the simple process of immersion or dip coating, the substrate is dipped into a bath of the coating, which is normally of a low viscosity to enable the coating to run back into the bath as the substrate emerges.

In the metering rod coating process, an excess of the coating is deposited onto the substrate as it passes over the bath roller. The wire-wound metering rod, sometimes known as a Meyer Bar, allows the desired quantity of the coating to remain on the substrate. The quantity is determined by the diameter of the wire used on the rod.

In the slot die process, the coating is squeezed out by gravity or under pressure through a slot and onto the substrate. If the coating is 100% solids, the process is termed "Extrusion" and in this case, the line speed is frequently much faster than the speed of the extrusion. This enables coatings to be considerably thinner than the width of the slot.

The gap or knife over roll process relies on a coating being applied to the substrate which then passes through a "gap" between a "knife" and a support roller. As the coating and substrate pass through, the excess is scraped off.

Air knife coating is where the coating is applied to the substrate and the excess is "blown off" by a powerful jet from the air knife. This procedure is useful for aqueous coatings.

In the curtain coating process, a bath with a slot in the base allows a continuous curtain of the coating to fall into the gap between two conveyors. The object to be coated is passed along the conveyor at a controlled speed and so receives the coating on its upper face.

**Drying the Film**

While the proper viscosity, uniformity in mixture and stable suspension of particles, and casting method are important in the initial steps of forming the film to promote uniformity, the method of drying the wet film is also important. Although these parameters and properties assist uniformity initially, a controlled rapid drying process ensures that the uniformity will be maintained until the film is dry. A controlled drying process is particularly important when, in the absence of a viscosity increasing composition or a composition in which the viscosity is controlled, for example by the selection of the polymer, the components within the film may have an increased tendency to aggregate or conglomerate. An alternative method of forming a film with an accurate dosage, that would not necessitate the controlled drying process, would be to cast the films on a predetermined well. With this method, although the components may aggregate, this will not result in the migration of the active to an adjacent dosage form, since each well may define the dosage unit per se.

When a controlled or rapid drying process is desired, this may be through a variety of methods. A variety of methods may be used including those that require the application of heat. The liquid carriers are removed from the film in a. manner such that the uniformity, or more specifically, the non-self-aggregating uniform heterogeneity, that is obtained in the wet film is maintained.

Desirably, the film is dried from the bottom of the film to the top of the film, Substantially no air flow is present across the top of the film during its initial setting period, during which a solid, visco-elastic structure is formed. This can take place within the first few minutes, e.g., the first ½ minute to the first 4 minutes of the drying process. Controlling the drying in this manner, prevents the destruction and reformation of the film's top surface, which results from conventional drying methods. This is accomplished by forming the film and placing it on the top side of a surface having top and bottom sides . Then, heat is initially applied to the bottom side of the film to provide the necessary energy to evaporate or otherwise remove the liquid carrier. The films dried in this manner dry more quickly and evenly as compared to air-dried films, or those dried by conventional drying means. In contrast to an air-dried film that dries first at the top and edges, the films dried by applying heat to the bottom dry simultaneously at the center as well as at the edges., This also prevents settling of ingredients that occurs with films dried by conventional means

The temperature at which the films are dried is 100°C or less, desirably 90°C on less, and most desirably 80°C or less.

Another method of controlling the drying process, which may be used done or in combination with other controlled methods as disclosed above includes controlling and modifying the humidity within the drying apparatus where the film is being dried In this manner, the premature drying of the top surface of the film is avoided.

A specific example of an appropriate drying method is that disclosed by Magoon. Magoon is specifically directed toward a method of drying fruit pulp. However, the present inventors have adapted this process toward the preparation of thin films,

The method and apparatus of Magoon are based on an interesting property of waster Although water transmits energy by conduction and convection both within and to its surroundings, water only radiates energy within and to water Therefore, the apparatus of Magoon includes a surface onto which the fruit pulp is placed that is transparent to infrared radiation. The underside of the surface is in contact with a temperature controlled water bath. The water bath temperature is desirably controlled at a temperature slightly below the boiling temperature of water. When the wet fruit pulp is placed on the surface of the apparatus, this creates a "refractance window." This means that infrared energy is permitted to radiate through the surface only to the area on the surface occupied by the fruit pulp, and only until the fruit pulp is dry. The apparatus of Magoon provides the films of the present invention with an efficient drying time reducing the instance of aggregation of'the components of the film,

The films may initially have a thickness of 500 $\mu$m to 1,500 $\mu$m, or about 20 mils to about 60 mils, and when dried have a thickness from 3 $\mu$m to 250 $\mu$m, or about 0.1mils to about 10mils. Desirably, the dried films will have a thickness of about 50.8 $\mu$m (2 mils) to 203.2$\mu$m (8 mils) and more desirably, from 76.2$\mu$m (3 mils) to 152.4 $\mu$m (6 mils).

The wet film is then dried using controlled bottom drying or controlled microwave drying, desirably in the absence of external air currents or heat on the top (exposed) surface of the film 48 as described herein Controlled bottom dying or controlled microwave dying advantageously allows for vapor release from the film without the disadvantages of the prior art. Conventional convection air drying from the top is not employed because it initiates drying at the top uppermost portion of the film, thereby forming a barrier against fluid flow, such as the evaporative vapors, and thermal flow, such as the thermal energy for crying Such dried upper portions serve as a barrier to further vapor release as the portions beneath are dried, which results in non-uniform films. As previously mentioned some top air flow can be used to aid the drying of the films of the present invention, but it must not create a condition that would cause particle movement or a rippling effect in the film, both of which would results in non-uniformity If top air is employed, it is balanced with the bottom air drying to avoid non-uniformity and prevent film lift-up on the carrier belt A balance top and bottom air flow may be suitable where the bottom air flow functions as the major source of drying and the top air flow is the minor source of drying The advantage of some top air flow is to move the exiting vapors away from the film thereby aiding in the overall drying process. The use of any top air flow or top drying, however, must be balanced by a number of factors including, but not limited, to rheological properties of the composition and mechanical aspects of the processing. Any top fluid flow, such as air, also must not overcome the inherent viscosity of the film-forming composition. In other words, the top air flow cannot break, distort or otherwise physically disturb the surface of the composition. Moreover, air velocities are desirably below the yield values of the film, i.e., below any force level that can move the liquids in the film-forming compositions. For thin or low viscosity compositions, low air velocity must be used. For thick or high viscosity compositions, higher air velocities may be used. Furthermore, air velocities are desirable low so as to avoid any lifting or other movement of the film formed from the compositions.

Moreover, the films of the present invention may contain particles that are sensitive to temperature, such as flavors, which may be volatile, or drugs, which may have a low degradation temperature. In such cases, the drying temperature may be decreased while increasing the drying time to adequately dry the uniform films of the present invention. Furthermore, bottom drying also tends to result in a lower internal film temperature as compared to top drying. In bottom drying, the evaporating vapors more readily carry heat away from the film as compared to top drying which lowers the internal film temperature. Such lower internal film temperatures often result in decreased drug degradation and decreased loss of certain volatiles, such as flavors.

Furthermore, particles or particulates may be added to the film-forming composition or matrix after the composition or matrix is cast into a film. For example, particles may be added to the film 42 prior to the drying of the film 42. Particles may be controllably metered to the film and disposed onto the film through a suitable technique, such as through the use of a doctor blade (not shown) which is a device which marginally or softly touches the surface of the film and controllably disposes the particles onto the film surface. Other suitable, but non-limiting, techniques include the use of an additional roller to place the particles on the film surface, spraying the particles onto the film surface, and the like. The particles may be placed on either or both of the opposed film surfaces, i.e., the top and/or bottom film surfaces. Desirably, the particles are securably disposed onto the film, such as being embedded into the film. Moreover, such particles are desirably not fully encased or fully embedded into the film, but remain exposed to the surface of the film, such as in the case where the particles are partially embedded or partially encased.

The particles may be any useful organoleptic agent, cosmetic agent, pharmaceutical agent, or combinations thereof. Desirably, the pharmaceutical agent is a taste-masked or a controlled-release pharmaceutical agent. Useful organoleptic agents include flavors and sweeteners. Useful cosmetic agents include breath freshening or decongestant agents, such as menthol, including menthol crystals

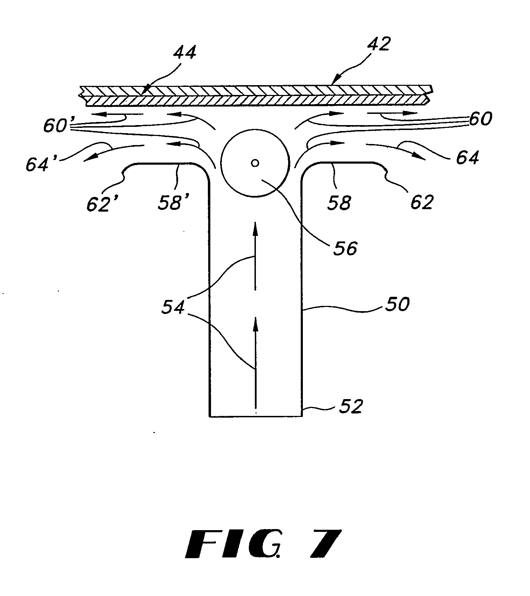

Although the inventive process is not limited to any particular apparatus for the above-described desirable drying, one particular useful drying apparatus 50 is depicted in Figure 7. Drying apparatus 50 is a nozzle arrangement for directing hot fluid, such as but not limited to hot air, towards the bottom of the film 42 which is disposed on substrate 44. Hot air enters the entrance end 52 of the drying apparatus and travels vertically upward, as depicted by vectors 54, towards air deflector 56.. The air deflector 56 redirects the air movement to minimize upward force on the film 42.. As depicted in Figure 7, the air is tangentially directed, as indicated by vectors 60 and 60', as the air passes by air deflector 56 and enters and travels through chamber portions 58 and 58' of the drying apparatus 50 With the hot air flow being substantially tangential to the film 42, lifting of the film as it is being dried is thereby minimized. While the air deflector 56 is depicted as a roller, other devices and geometries for deflecting air or hot fluid may suitable be used.. Furthermore, the exit ends 62 and 62' of the drying apparatus 50 are flared downwardly Such downward flaring provides a downward force or downward velocity vector, as indicated by vectors 64 and 64', which tend to provide a pulling or drag effect of the film 42 to prevent lifting of the film 42 Lifting of the film 42 may not only result in non-uniformity in the film or otherwise, but may also result in non-controlled processing of the film 42 as the film 42 and/or substrate 44 lift away from the processing equipment.

Monitoring and control of the thickness of the film also contributes to the production of a uniform film by providing a film of uniform thickness. The thickness of the film may be monitored with gauges such as Beta Gauges. A gauge may be coupled to another gauge at the end of the drying apparatus, i.e.. drying oven or tunnel, to communicate through feedback loops to control and adjust the opening in the coating apparatus, resulting in control of uniform film thickness.

The film products are generally formed by combining a properly selected polymer and polar solvent, as well as any active ingredient or filler as desired Desuably, the solvent content of the combination is at least 30% by weight of the total combination. The matrix formed by this combination is formed into a film, desirably by roll coating, and then dried, desirably by a rapid and controlled drying process to maintain the uniformity of the film, more specifically, a non-self aggregating uniform heterogeneity.. The resulting film will desirably contain less than 10% by weight solvent, more desirably less than 8% by weight solvent, even more desirably less than 6% by weight solvent and most desirably less than 2%. The solvent may be water, a polar organic solvent including, but not limited to, ethanol, isopropanol, acetone, methylene chloride, or any combination thereof

It has also been unexpectedly discovered that high temperature fat materials, e g. MP, 55°C or greater, can be used to encapsulate dry particles before or after enteric coating, The drying process temperatures are sufficiently rapid and low, and evaporative cooling effect as a result of water vapor loss is sufficiently high enough, that the fat does not appreciably melt.

Consideration of the above discussed parameters, such as but not limited to rheology properties, viscosity, mixing method, casting method and drying method, also impact material selection for the different components of the present invention. Furthermore, such consideration with proper material selection provides the compositions of the present invention, including a pharmaceutical and/or cosmetic dosage form or film product having no more than a 10% variance of a pharmaceutical, and/or cosmetic active per unit area. In other words, the uniformity of the present invention is determined by the presence of no more than a 10% by weight of pharmaceutical and/or cosmetic variance throughout the matrix. Desirably, the variance is less than 5% by weight, less than 2% by weight, less than 1% by weight, or less than 0.5% by weight

## Uses of Thin Films

The thin films of the present invention are well suited for many uses. The high degree of uniformity of the components of the film makes them particularly well suited for incorporating pharmaceuticals. Furthermore, the polymers used in construction of the films may be chosen to allow for a range of disintegration times for the films. A variation or extension in the time over which a film will disintegrate may achieve control over the rate that the active is released, which may allow for a sustained release delivery system. In addition, the films may be used for the administration of an active to any of several body surfaces, especially those including mucous membranes, such as oral, anal, vaginal, ophthalmological, the surface of a wound, either on a skin surface or within a body such as during surgery, and similar surfaces.

The films may be used to orally administer an active. This is accomplished by preparing the films as described above and introducing them to the oral cavity of a mammal. This film may be prepared and adhered to a second or support layer from which it is removed prior to use, i.e. introduction to the oral cavity. An adhesive may be used to attach the film to the support or backing material which may be any of those known in the art, and is preferably not water soluble. If an adhesive is used, it will desirably be a food grade adhesive that is ingestible and does not alter the properties of the active. Mucoadhesive compositions are particularly useful. The film compositions in many cases serve as mucoadhesives themselves.

The films may be applied under or to the tongue of the mammal. When this is desired, a specific film shape, corresponding to the shape of the tongue may be preferred. Therefore the film may be cut to a shape where the side of the film corresponding to the back of the tongue will be longer than the side corresponding to the front of the tongue. Specifically, the desired shape may be that of a triangle or trapezoid. Desirably, the film will adhere to the oral cavity preventing it from being ejected from the oral cavity and permitting more of the active to be introduced to the oral cavity as the film dissolves.

Another use for the films of the present invention takes advantage of the films' tendency to dissolve quickly when introduce to a liquid. An active may be introduced to a liquid by preparing a film in accordance with the present invention, introducing it to a liquid, and allowing it to dissolve. This may be used either to prepare a liquid dosage form of an active, or to flavor a beverage.

The films of the present invention are desirably packaged in sealed, air and moisture resistant packages to protect the active from exposure oxidation, hydrolysis, volatilization and interaction with the environment. Referring to Figure 1, a packaged pharmaceutical dosage unit 10, includes each film 12 individually wrapped in a pouch or between foil and/or plastic laminate sheets 14. As depicted in Figure 2, the pouches 10,10' can be linked together with tearable or perforated joints 16. The pouches 10, 10'may be packaged in a roll as depicted in Figure 5 or stacked as shown in Figure 3 and sold in a dispenser 18 as shown in Figure 4. The dispenser may contain a full supply of the medication typically prescribed for the intended therapy, but due to the thinness of the film and package, is smaller and more convenient than traditional bottles used for tablets, capsules and liquids. Moreover, the films of the present invention dissolve instantly upon contact with saliva or mucosal membrane areas, eliminating the need to wash the dose down with water.

Desirably, a series of such unit doses are packaged together in accordance with the prescribed regimen or treatment, eg, a 10-90 day supply, depending on the particular therapy. The individual films can be packaged on a backing and peeled off for use.

### Rheology and Films Properties

For the purposes of the present invention the term non-self-aggregating uniform heterogeneity refers to the ability of the films of the present invention, which are formed from one or more components in addition to a polar solvent, to provide a substantially reduced occurrence of, i.e. little or no, aggregation or conglomeration of components within the film as is normally experienced when films are formed by conventional diying methods such as a high-temperature air-bath using a drying oven, drying tunnel, vacuum drier, or other such drying equipment. The term heterogeneity, as used in the present invention, includes films that will incorporate a single component, such as a polymer, as well as combinations of components, such as a polymer and an active. Uniform heterogeneity includes the substantial absence of aggregates or conglomerates as is common in conventional mixing and heat drying methods used to form films.

Furthermore, the films of the present invention have a substantially uniform thickness, which is also not provided by the use of conventional drying methods used for drying water-based polymer systems. The absence of a uniform thickness detrimentally affects uniformity of component distribution throughout the area of a given film

The film products of the present invention are produced by a combination of a properly selected polymer and a polar solvent, optionally including an active ingredient as well as other fillers known in the art These films provide a non-self-aggregating uniform heterogeneity of the components within them by utilizing a selected casting or deposition method and a controlled drying process. Examples of controlled drying processes include, but are not limited to, the use of the apparatus disclosed in U.S. Patent No. 4,631,837 to Magoon ("Magoon"), as well as hot air impingement across the bottom substrate and bottom heating plates. Another drying technique for obtaining the films of the present invention is controlled radiation drying, in the absence of uncontrolled air currents, such as infrared and radio frequency radiation (i.e. microwaves).

The objective of the drying process is to provide a method of drying the films that avoids complications, such as the noted "rippling" effect, that are associated with conventional drying methods and which initially dry the upper surface of the film, trapping moisture inside. In conventional oven drying methods, as the moisture trapped inside subsequently evaporates, the top surface is altered by being ripped open and then reformed. These complications are avoided by the present invention, and a uniform film is provided by drying the bottom surface of the film first or otherwise preventing the formation of polymer film formation (skin) on the top surface of the film prior to drying the depth of the film. This may be achieved by applying heat to the bottom surface of the film with substantially no top air flow, or alternatively by the introduction of controlled microwaves to evaporate the water or other polar solvent within the film, again with substantially no top air flow. Yet alternatively, drying may be achieved by using balanced fluid flow, such as balanced air flow, where the bottom and top air flows are controlled to provide a uniform film. In such a case, the air flow directed at the top of the film should not create a condition which would cause movement of particles present in the wet film, due to forces generated by the air currents. Additionally, air currents directed at the bottom of the film should desirably be controlled such that the film does not lift up due to forces from the air. Uncontrolled air currents, either above or below the film, can create non-unifomlity in the final film products. The humidity level of the area surrounding the top surface may also be appropriately adjusted to prevent premature closure or skinning of the polymer surface.

This manner of drying the films provides several advantages. Among these are the faster drying times and a more uniform surface of the film, as well as uniform distribution of components for any given area in the film. In addition, the faster drying time allows viscosity to quickly build within the film, further encouraging a uniform distribution of components and decrease in aggregation of components in the final film product. Desirably, the drying of the film will occur within about ten minutes or fewer, or more desirably within about five minutes or fewer.

The present invention yields exceptionally uniform film products when attention is paid to reducing the aggregation of the compositional components. By avoiding the introduction of and eliminating excessive air in the mixing process, selecting polymers and solvents to provide a controllable viscosity and by drying the film in a rapid manner from the bottom up, such films result.

The products and processes of the present invention rely on the interaction among various steps of the production of the films in order to provide films that substantially reduce the self-aggregation of the components within the films. Specifically, these steps include the particular method used to form the film, making the composition mixture to prevent air bubble inclusions, controlling the viscosity of the film forming composition and the method of drying the film. More particularly, a greater viscosity of components in the mixture is particularly useful when the active is not soluble in the selected polar solvent in order to prevent the active from settling out. However, the viscosity must not be too great as to hinder or prevent the chosen method of casting, which desirably includes reverse roll coating due to its ability to provide a film of substantially consistent thickness.

In addition to the viscosity of the film or film-forming components or matrix, there are other considerations taken into account by the present invention for achieving desirable film uniformity. For example, stable suspensions are achieved which prevent solid (such as drug particles) sedimentation in non-colloidal applications. One approach provided by the present invention is to balance the density of the particulate ($\rho_p$) and the liquid phase ($\rho_1$) and increase the viscosity of the liquid phase ($\mu$). For an isolated particle, Stokes law relates the terminal settling velocity (Vo) of a rigid spherical body of radius (r) in a viscous fluid, as follows:

$$V_o = (2gr^r)(\rho_p - \rho_l)/9\mu$$

At high particle concentrations, however, the local particle concentration will affect the local viscosity and density. The viscosity of the suspension is a strong function of solids volume fraction, and particle-particle and particle-liquid interactions will further hinder settling velocity.

Stokian analyses has shown that the incorporation of a third phase, dispersed air or nitrogen, for example, promotes suspension stability. Further, increasing the number of particles leads to a hindered settling effect based on the solids volume fraction. In dilute particle suspensions, the rate of sedimentation, v, can be expressed as:

$$v/V_o = 1/(1 + \kappa\varphi)$$

where $\kappa$ = a constant, and $\varphi$ is the volume fraction of the dispersed phase. More particles suspended in the liquid phase results in decreased velocity. Particle geometry is also an important factor since the particle dimensions will affect particle-particle flow interactions.

Similarly, the viscosity of the suspension is dependent on the volume fraction of dispersed solids. For dilute suspensions of non-interaction spherical particles, an expression for the suspension viscosity can be expressed as:

$$\mu/\mu_o = 1 + 2.5\phi$$

where $\mu_o$ is the viscosity of the continuous phase and $\phi$ is the solids volume fraction. At higher volume fractions, the viscosity of the dispersion can be expressed as

$$\mu/\mu_o = 1 + 2.5\varphi + C_1\varphi^2 + C_2\varphi^3 + \ldots..$$

where C is a constant.

The viscosity of the liquid phase is critical and is desirably modified by customizing the liquid composition to a viscoelastic non-Newtonian fluid with low yield stress values. This is the equivalent of producing a high viscosity continuous phase at rest. Formation of a viscoelastic or a highly structured fluid phase provides additional resistive forces to particle sedimentation. Further, flocculation or aggregation can be controlled minimizing particle-particle interactions. The net effect would be the preservation of a homogeneous dispersed phase.

The addition of hydrocolloids to the aqueous phase of the suspension increases viscosity, may produce viscoelasticity and can impart stability depending on the type of hydrocolloid, its concentration and the particle composition, geometry, size, and volume fraction. The particle size distribution of the dispersed phase needs to be controlled by selecting the smallest realistic particle size in the high viscosity medium, i.e., $<500\mu m$. The presence of a slight yield stress or elastic body at low shear rates may also induce permanent stability regardless of the apparent viscosity. The critical particle diameter can be calculated from the yield stress values. In the case of isolated spherical particles, the maximum shear stress developed in settling through a medium of given viscosity can be given as

$$\tau_{max} = 3V\mu/2r$$

For pseudoplastic fluids, the viscosity in this shear stress regime may well be the zero shear rate viscosity at the Newtonian plateau.

A stable suspension is an important characteristic for the manufacture of a pre-mix composition which is to be fed into the film casting machinery film, as well as the maintenance of this stability in the wet film stage until sufficient drying has occurred to lock-in the particles and matrix into a sufficiently solid form such that uniformity is maintained. For viscoelastic fluid systems, a rheology that yields stable suspensions for extended time period, such as 24 hours, must be balanced with the requirements of highspeed film casting operations. A desirable property for the films is shear thinning or pseudoplasticity, whereby the viscosity decreases with increasing shear rate. Time dependent shear effects such as thixotropy are also advantageous. Structural recovery and shear thinning behavior are important properties, as is the ability for the fihn to self-level as it is formed.

The rheology requirements for the inventive compositions and films are quite severe. This is due to the need to produce a stable suspension of particles, for example 30-60 wt%, in a viscoelastic fluid matrix with acceptable viscosity values throughout a broad shear rate range. During mixing, pumping, and film casting, shear rates in the range of 10 - $10^5$ sec.$^{-1}$ may be experienced and pseudoplasticity is the preferred embodiment.

In film casting or coating, rheology is also a defining factor with respect to the ability to form films with the desired uniformity. Shear viscosity, extensional viscosity, viscoelasticity, structural recovery will influence the quality of the film. As an illustrative example, the leveling of shear-thinning pseudoplastic fluids has been derived as

$$\alpha^{(n-1/n)} = \alpha_o^{(n-1/n)} - ((n-1)/(2n-1))(\tau/K)^{1/n} (2\pi/\lambda)^{(3+n)/n}h^{(2n+1)/n}t$$

where $\alpha$ is the surface wave amplitude, $\alpha_o$ is the initial amplitude, $\lambda$ is the wavelength of the surface roughness, and both "n" and "K" are viscosity power law indices. In this example, leveling behavior is related to viscosity, increasing as n decreases, and decreasing with increasing K.

Desirably, the films or film-forming compositions of the present invention have a very rapid structural recovery, i.e. as the film is formed during processing, it doesn't fall apart or become discontinuous in its structure and compositional uniformity. Such very rapid structural recovery retards particle settling and sedimentation. Moreover, the films or film-forming compositions of the present invention are desirably shear-thinning pseudoplastic fluids. Such fluids with consideration of properties, such as viscosity and elasticity, promote thin film formation and uniformity.

Thus, uniformity in the mixture of components depends upon numerous variables, As described herein, viscosity of the components, the mixing techniques and the rheological properties of the resultant mixed composition and wet casted film are important aspects of the present invention. Additionally, control of particle size and particle shape are further considerations. Desirably, the size of the particulate a particle size of 150 microns or less, for example 100 microns or less. Moreover, such particles may be spherical, substantially spherical, or non-spherical, such as irregularly shaped particles or ellipsoidally shaped particles. Ellipsoidally shaped particles or ellipsoids are desirable because of their ability to maintain uniformity in the film forming matrix as they tend to settle to a lesser degree as compared to spherical particles

Although a variety of different polymers may be used, it is desired to select polymers to provide a desired viscosity of the mixture prior to drying, For example, if the active or other components are not soluble in the selected solvent, a polymer that will provide a greater viscosity is desired to assist in maintaining uniformity. On the other hand, if the components are soluble in the solvent, a polymer that provides a lower viscosity may be preferred.

The polymer plays an important role in affecting the viscosity of the film. Viscosity is one property of a liquid that controls the stability of the active in an emulsion, a colloid or a suspension Generally the viscosity of the matxix will vary from 400 cps ("cps" or "centipoise") to 100,000 cps, preferably from 800 cps to 60,000 cps, and most preferably from 1,000 cps to 40,000 cps Desirably, the viscosity of the film-forming matrix will rapidly increase upon initiation of the drying process.

The viscosity may be adjusted based on the selected active depending on the other components within the matrix, For example, if the component is not soluble within the selected solvent, a proper viscosity may be selected to prevent the component from settling which would adversely affect the uniformity of the resulting film. The viscosity may be adjusted in different ways. To increase viscosity of the film matrix, the polymer may be chosen of a higher molecular weight or crosslinkers may be added, such as salts of calcium, sodium and potassium. The viscosity may also be adjusted by adjusting the temperature or by adding a viscosity increasing component. Components that will increase the viscosity or stabilize the emulsion/suspension include higher molecular weight polymers and polysaccharides and gums, which include without limitation, alginate, carrageenan, hydroxypropyl methyl cellulose, locust bean gum, guar gum, xanthan gum, dextran, gum arabic, gellan gum and combinations thereof.

## Film Component Mixing:

A number of techniques may be employed in the mixing stage to prevent bubble inclusions in the final film. To provide a composition mixture with substantially no air bubble formation in the final product, anti-foaming or surface-tension reducing agents are employed. Additionally, the speed of the mixture is desirably controlled to prevent cavitation of the mixture in a manner which pulls air into the mix. Finally, air bubble reduction can further be achieved by allowing the mix to stand for a sufficient time for bubbles to escape prior to drying the film. Desirably, the inventive process first forms a masterbatch of film-forming components without active ingredients such as drug particles or volatile materials such as flavor oils. The actives are added to smaller mixes of the masterbatch just prior to casting. Thus, the masterbatch pre-mix can be allowed to stand for a longer time without concern for instability in drug or other ingredients.

When the matrix is formed including the film-forming polymer and polar solvent in addition to any additives and the active ingredient, this may be done in a number of steps. For example, the ingredients may all be added together or a pre-mix may be prepared. The advantage of a pre-mix is that all ingredients except for the active may be combined in advance, with the active added just prior to formation of the film. This is especially important for actives that may degrade with prolonged exposure to water, air or another polar solvent.

Figure 6 shows an apparatus 20 suitable for the preparation of a pre-mix, addition of an active and subsequent formation of a film. The pre-mix or master batch 22, which includes the film-forming polymer, polar solvent, and any other additives except a drug active is added to the master batch feed tank 24. The components for pre-mix or master batch 22 are desirably formed in a mixer (not shown) prior to their addition into the master batch feed tank 24. Then a pre-determined amount of the master batch is controllably fed via a first metering pump 26 and control valve 28 to either or both of the first and second mixers, 30, 30’. The present invention, however, is not limited to the use of two mixers, 30, 30’, and any number of mixers may suitably be used. Moreover, the present invention is not limited to any particular sequencing of the mixers 30, 30’, such as parallel sequencing as depicted in Figure 6, and other sequencing or arrangements of mixers, such as series or combination of parallel and series, may suitably be used. The required amount of the drug or other ingredient, such as a flavor, is added to the desired mixer through an opening, 32, 32’, in each of the mixers, 30, 30’. Desirably, the residence time of the pre-mix or master batch 22 is minimized in the mixers 30, 30’. While complete dispersion of the drug into the pre-mix or master batch 22 is desirable, excessive residence times may result in leaching or dissolving of the drug, especially in the case for a soluble drug. Thus, the mixers 30, 30’ are often smaller, i.e. lower residence times, as compared to the primary mixers (not shown) used in forming the pre-mix or master batch 22. After the drug has been blended with the master batch pre-mix for a sufficient time to provide a uniform matrix, a specific amount of the uniform matrix is then fed to the pan 36 through the second metering pumps, 34, 34’. The metering roller 38 determines the thickness of the film 42 and applies it to the application roller. The film 42 is finally formed on the substrate 44 and carried away via the support roller 46.

## Forming the Film

The films of the present invention must be formed into a sheet prior to drying. After the desired components are combined to form a multi-component matrix, including the polymer, water, and an active or other components as desired, the combination is formed into a sheet or film, by any method known in the art such as extrusion, coating, spreading, casting or drawing the multi-component matrix. If a multi-layered film is desired, this may be accomplished by co-extruding more than one combination of components which may be of the same or different composition. A multi-layered film may also be achieved by coating, spreading, or casting a combination onto an already formed film layer.

Although a variety of different film-forming techniques may be used, it is desirable to select a method that will provide a flexible film, such as reverse roll coating. The flexibility of the film allows for the sheets of film to be rolled and transported for storage or prior to being cut into individual dosage forms. Desirably, the films will also be self-supporting or in other words able to maintain their integrity and structure in the absence of a separate support. Furthermore, the films of the present invention may be selected of materials that are edible or ingestible.

Coating or casting methods are particularly useful for the purpose of forming the films of the present invention. Specific examples include reverse roll coating, gravure coating, immersion or dip coating, metering rod or meyer bar coating, slot die or extrusion coating, gap or knife over roll coating, air knife coating, curtain coating, or combinations thereof, especially when a multi-layered film is desired.

Roll coating, or more specifically reverse roll coating, is particularly desired when forming films in accordance with the present invention. This procedure provides excellent control and uniformity of the resulting films, which is desired in the present invention. In this procedure, the coating material is measured onto the applicator roller by the precision setting of the gap between the upper metering roller and the application roller below it. The coating is transferred from the application roller to the substrate as it passes around the support roller adjacent to the application roller. Both three roll and four roll processes are common.

The gravure coating process relies on an engraved roller running in a coating bath, which fills the engraved dots or lines of the roller with the coating material. The excess coating on the roller is wiped off by a doctor blade and the coating is then deposited onto the substrate as it passes between the engraved roller and a pressure roller.

Offset Gravure is common, where the coating is deposited on an intermediate roller before transfer to the substrate.

In the simple process of immersion or dip coating, the substrate is dipped into a bath of the coating, which is normally of a low viscosity to enable the coating to run back into the bath as the substrate emerges.

In the metering rod coating process, an excess of the coating is deposited onto the substrate as it passes over the bath roller. The wire-wound metering rod, sometimes known as a Meyer Bar, allows the desired quantity of the coating to remain on the substrate. The quantity is determined by the diameter of the wire used on the rod.

In the slot die process, the coating is squeezed out by gravity or under pressure through a slot and onto the substrate. If the coating is 100% solids, the process is termed "Extrusion" and in this case, the line speed is frequently much faster than the speed of the extrusion. This enables coatings to be considerably thinner than the width of the slot.

The gap or knife over roll process relies on a coating being applied to the substrate which then passes through a "gap" between a "knife" and a support roller. As the coating and substrate pass through, the excess is scraped off.

Air knife coating is where the coating is applied to the substrate and the excess is "blown off' by a powerful jet from the air knife. This procedure is useful for aqueous coatings.

In the curtain coating process, a bath with a slot in the base allows a continuous curtain of the coating to fall into the gap between two conveyors. The object to be coated is passed along the conveyor at a controlled speed and so receives the coating on its upper face.

## Drying the Film

While the proper viscosity, uniformity in mixture and stable suspension of particles, and casting method are important in the initial steps of forming the film to promote uniformity, the method of drying the wet film is also important. Although these parameters and properties assist uniformity initially, a controlled rapid drying process ensures that the uniformity will be maintained until the film is dry. A controlled drying process is particularly important when, in the absence of a viscosity increasing composition or a composition in which the viscosity is controlled, for example by the selection of the polymer, the components within the film may have an increased tendency to aggregate or conglomerate. An alternative method of forming a film with an accurate dosage, that would not necessitate the controlled drying process, would be to cast the films on a predetermined well. With this method, although the components may aggregate, this will not result in the migration of the active to an adjacent dosage form, since each well may define the dosage unit per se.

When a controlled or rapid drying process is desired, this may be through a variety of methods. A variety of methods may be used including those that require the application of heat. The liquid carriers are removed from the film in a manner such that the uniformity, or more specifically, the non-self aggregating uniform heterogeneity, that is obtained in the wet film is maintained.

Desirably, the film is dried from the bottom of the film to the top of the film. Substantially no air flow is present across the top of'the film during its initial setting period, during which a solid, visco-elastic structure is formed This can take place within the first few minutes, e g. the first ½ minute to the first 4 minutes of the drying process. Controlling the drying in this manner, prevents the destruction and reformation of the film's top surface, which results from conventional drying methods. This is accomplished by forming the film and placing it on the top side of a surface having top and bottom sides Then, heat is initially applied to the bottom side of the film to provide the necessary energy to evaporate or otherwise remove the liquid carrier. The films dried in this manner dry more quickly and evenly as compared to air dried films, or those dried by conventional drying means. In contrast to an air-dried film that dries first at the top and edges, the films dried by applying heat to the bottom dry simultaneously at the center as well as at the edges This also prevents settling of ingredients that occurs with films dried by conventional means,

The temperature at which the films are dried is 100°C or less, desirably 90°C or less, and most desirably 80°C or less.

Another method of controlling the drying process, which may be used alone or in combination with other controlled methods as disclosed above includes controlling and modifying the humidity within the drying apparatus where the film is being dried. In this manner, the premature drying of the top surface of the film is avoided.

A specific example of an appropriate drying method is that disclosed by Magoon Magoon is specifically directed toward a method of drying fruit pulp. However, the present inventors have adapted this process toward the preparation of thin films..

The method and apparatus of Magoon are based on an interesting property of water Although water transmits energy by conduction and convection both within and to its surroundings, water only radiates energy within and to water Therefore, the apparatus of Magoon includes a surface onto which the fruit pulp is placed that is transparent to infrared radiation The underside of the surface is in contact with a temperature controlled water bath. The water bath temperature is desirably controlled at a temperature slightly below the boiling temperature of water. When the wet fruit pulp is placed on the surface of the apparatus, this creates a "refractance window." This means that infrared energy is permitted to radiate through the surface only to the area on the surface occupied by the fruit pulp, and only until the fruit pulp is dry. The apparatus of Magoon provides the films of the present invention with an efficient drying time reducing the instance of aggregation of the components of the film.

The films may initially have a thickness of 500 μm to 1,500 μm, or about 20 mils to about 60 mils, and when dried have a thickness from 3 μm to 250 μm, or about 0.1mils to about 10mils Desirably, the dried films will have a thickness of 50.8 μm (2 mils) to 203.2 μm (8 mils), and more desirably, from 76.2 μm (3 mils) to 152.4 μm (6 mils).

The wet film is then dried using controlled bottom drying or controlled microwave drying, desirably in the absence of external air currents or heat on the top (exposed) surface of the film 48 as described herein. Controlled bottom drying or controlled microwave drying advantageously allows for vapor release from the film without the disadvantages of the prior art Conventional convection air drying from the top is not employed because it initiates drying at the top uppermost portion of the film, thereby forming a barrier against fluid flow, such as the evaporative vapors, and thermal flow, such as the thermal energy for drying. Such dried upper portions serve as a barrier to further vapor release as the portions beneath are dried, which results in non-uniform films. As previously mentioned some top air flow can be used to aid the drying of the films of the present invention, but it must not create a condition that would cause particle movement or a rippling effect in the film, both of which would result in non-uniformity If top air is employed, it is balanced with the bottom air drying to avoid non-uniformity and prevent film lift-up on the carrier belt A balance top and bottom air flow may be suitable where the bottom air flow functions as the major source of drying and the top air flow is the minor source of drying. The advantage of'some top air flow is to move the exiting vapors away from the film thereby aiding in the overall drying process. The use of any top air flow or top drying, however, must be balanced by a number of factors including, but not limited, to rheological properties of the composition and mechanical aspects of the processing. Any top fluid flow, such as air, also must not overcome the inherent viscosity of the film-forming composition. In other words, the top air flow cannot break, distort or otherwise physically disturb the surface of the composition. Moreover, air velocities are desirably below the yield values of the film, i.e., below any force level that can move the liquids in the film-forming compositions. For thin or low viscosity compositions, low air velocity must be used. For thick or high viscosity compositions, higher air velocities may be used. Furthermore, air velocities are desirable low so as to avoid any lifting or other movement of the film formed from the compositions.

Moreover, the films of the present invention may contain particles that are sensitive to temperature, such as flavors, which may be volatile, or drugs, which may have a low degradation temperature. In such cases, the drying temperature may be decreased while increasing the drying time to adequately dry the uniform films of the present invention. Furthermore, bottom drying also tends to result in a lower internal film temperature as compared to top drying. In bottom drying, the evaporating vapors more readily carry heat away from the film as compared to top drying which lowers the internal film temperature. Such lower internal film temperatures often result in decreased drug degradation and decreased loss of certain volatiles, such as flavors.

Furthermore, particles or particulates may be added to the film-forming composition or matrix after the composition or matrix is cast into a film. For example, particles may be added to the film 42 prior to the drying of the film 42. Particles may be controllably metered to the film and disposed onto the film through a suitable technique, such as through the use of a doctor blade (not shown) which is a device which marginally or softly touches the surface of the film and controllably disposes the particles onto the film surface. Other suitable, but non-limiting, techniques include the use of an additional roller to place the particles on the film surface, spraying the particles onto the film surface, and the like. The particles may be placed on either or both of the opposed film surfaces, i.e., the top and/or bottom film surfaces. Desirably, the particles are securably disposed onto the film, such as being embedded into the film. Moreover, such particles are desirably not fully encased or fully embedded into the film, but remain exposed to the surface of the film, such as in the case where the particles are partially embedded or partially encased.

The particles may be any useful organoleptic agent, cosmetic agent, pharmaceutical agent, or combinations thereof. Desirably, the pharmaceutical agent is a taste-masked or a controlled-release pharmaceutical agent. Useful organoleptic agents include flavors and sweeteners. Useful cosmetic agents include breath freshening or decongestant agents, such as menthol, including menthol crystals.

Although the inventive process is not limited to any particular apparatus for the above-described desirable drying, one particular useful drying apparatus 50 is depicted in Figure 7. Drying apparatus 50 is a nozzle arrangement for directing hot fluid, such as but not limited to hot air, towards the bottom of the film 42 which is disposed on substrate 44. Hot air enters the entrance end 52 of the drying apparatus and travels vertically upward, as depicted by vectors 54, towards air deflector 56 The air deflector 56 redirect the air movement to minimize upward force on the film 42 As depicted in Figure 7, the air is tangentially directed, as indicated by vectors 60 and 60', as the air passes by air deflector 56 and enters and travels through chamber portions 58 and 58' of the drying apparatus 50. With the hot air flow being substantially tangential to the film 42, lifting of the film as it is being dried is thereby minimized. While the air deflector 56 is depicted as a roller, other devices and geometries for deflecting air or hot fluid may suitable be used. Furthermore, the exit ends 62 and 62' of the drying apparatus 50 are flared downwardly. Such downward flaring provides a downward force or downward velocity vector, as indicated by vectors 64 and 64', which tend to provide a pulling or drag effect of the film 42 to prevent lifting of the film 42. Lifting of the film 42 may not only result in non-uniformity in the film or otherwise, but may also result in non-controlled processing of the film 42 as the film 42 and/or substrate 44 lift away from the processing equipment.

Monitoring and control of the thickness of the film also contributes to the production of a uniform film by providing a film of uniform thickness. The thickness of the film may be monitored with gauges such as Beta Gauge, A gauge may be coupled to another gauge at the end of the drying apparatus, i.e. drying oven or tunnel, to communicate through feedback loops to control and adjust the opening in the coating apparatus, resulting in control of uniform film thickness.

The film products are generally formed by combining a properly selected polymer and polar solvent, as well as any active ingredient or filler as desired Desirably, the solvent content of the combination is at least 30% by weight of the total combination. The matrix formed by this combination is formed into a films, desirably by roll coating, and then dried, desirably by a rapid and controlled drying process to maintain the uniformity of the film, more specifically, a non-self-aggregating uniform heterogeneity. The resulting film will desirably contain less than 10% by weight solvent, more desirably less than 8% by weight solvent, even more desirably less than 6% by weight solvent and most desirably less than 2%. The solvent may be water, a polar organic solvent including, but not limited to, ethanol, isopropanol, acetone, methylene chloride, or any combination thereof.

It has also been unexpectedly discovered that high temperature fat materials, e.g. M.P. 55°C or greater, can be used to encapsulate dry particles before or after enteric coating. The drying process temperatures are sufficiently rapid and low, and evaporative cooling effect as a result of water vapor loss is sufficiently high enough, that the fat does not appreciably melt.

Consideration of the above discussed parameters, such as but not limited to rheology properties, viscosity, mixing method, casting method and drying method, also impact material selection for the different components of the present invention. Furthermore, such consideration with proper material selection provides the compositions of the present invention, including a pharmaceutical and/or cosmetic dosage form or film product having no more than a 10% variance of a pharmaceutical and/or cosmetic active per unit area. In other words, the uniformity of the present invention is determined by the presence of no more than a 10% by weight of pharmaceutical and/or cosmetic variance throughout the matrix. Desirably, the variance is less than 5% by weight, less than 2% by weight, less than 1% by weight, or less than 0.5% by weight.

The features and advantages of the present invention are more fully shown by the following examples which are provided for purposes of illustration.

**EXAMPLES**

**Illustrative Examples 1-8:**

A variety of films were produced using pullulan. As an insoluble active component, calcium carbonate was used

The compositions in Table 1 below were made in accordance with the process set forth in co-pending U.S. Application No 10/074,272. The resultant uncut film exhibited uniformity in content particularly with respect to the insoluble active, as well as unit doses of ¾" by 1" by 127 μm (5 mils) cut therefrom.

## TABLE 1

| Films Containing Pullulan/Water-Soluble Polymer Combinations With Insoluble Calcium Carbonate Active | | | | |
|---|---|---|---|---|
| | Grams (% by weight) | | | |
| Components | 1 | 2 | 3 | 4 |
| Pullulan[1] | 5.0 (15.5) | 10.0 (23.5) | 20.0 (42.1) | 15.0 (35.2) |
| Modified Cellulose | 3.0 (9.3) | -- | -- | -- |
| Starches | -- | 10.0 (23.5) | -- | -- |
| Carageenan Gum | -- | -- | 4.0 (8.4) | -- |
| Xantham Gum | -- | -- | -- | 3.0 (7.0) |
| Alginate | -- | -- | -- | -- |
| Polyvinyl-pyrrolidone | -- | -- | -- | -- |
| Loratidine | -- | -- | -- | -- |
| Calcium Carbonate | 10.0 (31.0) | 10.0 (23.5) | 10.0 (21.1) | 10.0 (23.5) |
| Mint Flavor | 2.5 (7.7) | 2.0 (4.7) | 2.0 (4.2) | 2.5 (5.9) |
| Artificial Sweeteners | 2.0 (6.2) | 1.5 (3.5) | 1.5 (3.2) | 2.0 (4.7) |
| Tween 80 | 3.0 (9.3) | 2.5 (5.9) | 2.5 (5.3) | 2.5 (5.9) |
| Antifoaming agent | 0.2 (0.6) | 0.2 (0.5) | 0.2 (0.4) | 0.2 (0.5) |
| Propylene Glycol | 4.0 (12.4) | 3.0 (7.0) | 3.5 (7.4) | 4.0 (9.4) |
| Water[1], Potable | 180 (8.0) | 80 (8.0) | 100 (8.0) | 120 (8.0) |

[1] The dried film was assumed to have a moisture content of about 6.0-8.0% by weight.

[2] Other non-limiting Gums/Food Polymers may be used in combination with Pullulan: Pectin, Carboxymethyl Cellulose, Hydroxypropyl Cellulose, Polyvinyl Alcohol, Gum Arabic, Polyacrylic Acid, Dextrim, Gelatin, Zein, Soy Protein Isolate, Whey Protein Isolate, Milk Casein and combinations thereof.

### TABLE 1 cont'd

| Films Containing Pullulan/Water-Soluble Polymer Combinations With Insoluble Calcium Carbonate Active | | | | |
|---|---|---|---|---|
| | Grams (% by weight) | | | |
| Components | 5 | 6 | 7 | 8 |
| Pullulan[1] | 25.0 (39.1) | 30.0 (8.7) | 20.0 (43.6) | 0.4 (1.6) |
| Modified Cellulose | -- | -- | 2.0 (4.4) | 2.0 (8.0) |
| Starches | -- | -- | -- | -- |
| Carageenan Gum | -- | -- | -- | -- |
| Xantham Gum | -- | -- | -- | -- |
| Alginate | 3.0 (4.6) | -- | -- | -- |
| Polyvinyl-pyrrolidone | -- | 8.0 (2.3) | -- | -- |
| Loratidine | -- | -- | 10.0 (21.8) | 12.0 (47.8) |
| Calcium Carbonate | 10.0 (15.4) | 10.0 (12.5) | -- | -- |
| Mint Flavor | 2.5 (3.9) | 2.0 (3.1) | 2.0 (4.4) | 1.5 (6.0) |
| Artificial Sweeteners | 2.0 (3.1) | 2.0 (3.1) | 2.0 (4.4) | 1.5 (6.0) |
| Tween 80 | 3.0 (4.6) | 2.5 (3.9) | 2.0 (4.4) | 1.5 (6.0) |
| Antifoaming agent | 0.2 (0.3) | 0.2 (0.3) | 0.2 (0.4) | 0.2 (0.8) |
| Propylene Glycol | 4.0 (6.2) | 4.0 (6.2) | 4.0 (8.7) | 4.0 (15.9) |
| Water[1], Potable | 140 (8.0) | 100 (8.0) | 150 (8.0) | 120 (8.0) |

[1] The dried film was assumed to have a moisture content of about 6.0-8.0% by weight

[2] Other non-limiting Gums/Food Polymers may be used in combination with Pullulan: Pectin, Carboxymethyl Cellulose, Hydroxypropyl Cellulose, Polyvinyl Alcohol, Gum Arabic, Polyacrylic Acid, Dextrim, Gelatin, Zein, Soy Protein Isolate, Whey Protein Isolate, Milk Casein and combinations thereof

### Illustrative Examples 9-11:

The following examples describe films and film-forming compositions that use an ethoxylated caster oil as a surfactant, or alternatively are free of surfactants, plasticizers and/or polyalcohols. Desirably, the films or film-forming compositions are essentially free of surfactants. Moreover, the films or film-forming compositions of the present invention are desirably formulated to be essentially free of surfactants. Furthermore, the films or film-forming compositions are desirably formulated to be essentially free of plasticizers Still furthermore, the films or film-forming compositions of the present invention are desirably formulated to be essentially free of polyalcohols. Moreover, the films or film-forming compositions are desirably formulated to be essentially free of surfactants and plasticizers Furthermore, the films or film-forming compositions are desirably formulated to be essentially free of surfactants, plasticizers and polyalcohols

EP 1 463 490 B1

**TABLE 2**

| Ingredient | (parts by wt.) 9 |
|---|---|
| POLYMERS: | |
| Hydroxypropylmethyl cellulose | 15.6 |
| Cornstarch[1] | 10.41 |
| Polyvinylpyrrolidone | 10.41 |
| Xanthan Gum | 1.14 |
| | |
| SURFACTANT[2]: | 2.0 |
| | |
| PLASTICIZER[3]: | 11.67 |
| | |
| ANTI-FOAM AGENT[4] | 2.44 |
| | |
| OTHER | |
| Spearmint Flavor | 10.43 |
| Loratadine (drug) | 16.62 |
| Calcium Carbonate | 5.54 |
| Sweetener | 9.36 |
| [1]Available from Grain Processing Corporation as Pure Cote B792 <br> [2]Ethoxylated caster oil, Cremophor® EL available from BASF <br> [3]Propylane Glycol <br> [4]Silicone Emulsion | |

The above ingredients were added at 30% to 70% water and stirred until polymers were fully hydrated which took 45 min The mix was then put under vacuum to eliminate entrapped air Vacuum was added in a steady manner starting at 500 mm and progressing up to 760 mm over 45 min.

After release of the vacuum, 6 grams of the liquid was added to a coating paper using a 200 micron spiral wound rod and a K Control Coater Model 101 (RK Print Coat Inst. Ltd.). The paper substrate onto which the coating was added was a silicone coated paper. The coated paper was then dried at 90°C until about 5% moisture remained. The formula coated and dried to a film thickness of approx. 60 microns and quickly dissolved in the mouth.

**TABLE 3**

| Ingredient | (parts by wt.) 10 |
|---|---|
| POLYMERS: | |
| Hydroxypropylmethyl cellulose | 15.6 |
| Cornstarch[1] | 10.41 |
| Polyvinylpyrrolidone | 10.41 |
| | |
| PLASTICIZER/SOLVENT[2]: | 22.1 |
| | |

(continued)

| Ingredient | (parts by wt.) 10 |
|---|---|
| ANTI-FOAM AGENT[3] | 2.44 |
| | |
| OTHER | |
| Raspberry Flavor | 0.3 |
| Calcium Carbonate[4] | 30.38 |
| Sweetener | 8.36 |
| [1]Available from Grain Processing Corporation as Pure Cote B792 [2]Propylene Glycol [3]Polydimethyl Siloxane Emulsion [4]Functioned to mimic drug loading | |

The above ingredients were added to water at 40% until a homogeneous suspension was made. Vacuum was added over 20 min. starting at 500 mm Hg. and ending at 660 mm Hg. until all air was removed from suspension. Film was made as described in prior experiments. The liquid coated the silicone release substrate and dried to a uniform flexible film. The film passed the 180° bend test without cracking and dissolved in the mouth.

**TABLE 4**

| Ingredient | (parts by wt.) 11 |
|---|---|
| POLYMERS: | |
| Hydroxypropylmethyl cellulose | 7.8 |
| Hydroxypropyl cellulose | 7.8 |
| | |
| ANTI-FOAM AGENT[1] | 0.75 |
| | |
| OTHER | |
| Peppermint & Bittermint Flavor | 2.25 |
| Tastemasking Flavor[2] | 0.3 |
| Calcium Carbonate[3] | 15.2 |
| Sweeteners | 0.9 |
| [1]Polydimethyl Siloxane Emulsion [2]Prosweet from Virginia Dave [3]Functioned to mimic drug loading | |

The above ingredients were added at 30% to 70% water and stirred until polymers were fully hydrated which took 20 min The mix was then put under vacuum to eliminate entrapped air Vacuum was added in a steady manner up to 760 mm over 35 min.

After release of the vacuum, the liquid was added to a coating paper using a 350 micron smooth bar and a K Control Coater Model 101 (RK Print Coat Inst Ltd). The paper substrate onto which the coating was added was a silicone coated paper. The coated paper was then dried at 90°C until about 4% moisture remained The formula coated and dried to a film. The film had an acceptable taste and quickly dissolved in the mouth The tastemasking flavor is an ingredient that effects the taste receptors to mask the receptors from registering a different, typical undesirable, taste. The film passed the 180° bend test without cracking and dissolved in the mouth

**Illustrative Examples 12-15:**

The following example 13 according to the present invention describes film-forming compositions analyzed for uniformity and stability Film-forming compositions were formed as shown below in Table 5.

**TABLES**

| Ingredient | (grams) | | | |
|---|---|---|---|---|
| | 12 | 13 | 14 | 15 |
| Pullulan | 7.0 | 7.0 | | |
| Hydroxypropylmethyl cellulose | | 7.0 | 7.0 | 10.5 |
| Hydroxypropyl cellulose | | | 7.0 | 3.5 |
| Polydimethylsiloxane emulsion | 0.14 | 0.14 | 0.14 | 0.14 |
| Water | 86 | 86 | 86 | 86 |

The above ingredients were stirred and mixed under vacuum to eliminate entrapped air. Seventy grams of each solution and five grams of glass beads were combined into four ounce jars and manually shook for about one minute to completely mix the glass beads into the solutions. The mixed samples were placed aside for ten minutes. After ten minutes a 10 ml sample was obtained from the top surface of each jar by use of a syringe.

The 10 ml samples were filtered through a funnel containing filter paper. The materials collected on the filter were washed with water to leave the glass beads on the paper. The filter papers with the glass beads were then dried for ten minutes at 90°C. The weight of the filter and the resulting glass beads were measured, as shown below in Table 6.

**TABLE 6**

| Ingredient | (grams) | | | |
|---|---|---|---|---|
| | 12 | 13 | 14 | 15 |
| Weight of filter paper and glass beads collected after drying | 0.332 | 0.509 | 0.839 | 0.831 |
| Initial weight of filter paper | 0.151 | 0.143 | 0.143 | 0.140 |
| Weight of glass beads collected | 0.181 | 0.366 | 0.696 | 0.691 |

Samples 14 and 15 contained considerably more glass beads that sample 12, which was made from pullulan. This indicated the significant particulate, i.e. glass beads, settling occurred with composition containing only pullulan as the polymer. The viscosity of pullulan is thus too low to provide a film-forming matrix that can maintain uniformity of particulates therein. Increasing the amount of other polymers, such as hydroxypropylmethyl cellulose, significantly increases the film stability and uniformity, as noted by the increased weight of the glass beads.

There have been described what are presently believed to be the certain desirable embodiments of the invention.

**Claims**

1. An ingestible water-soluble delivery system in the form of a film composition comprising a glucan, a water-soluble polymer comprising a cellulosic material, wherein said cellulosic material is selected from the group consisting of carboxymethyl cellulose, hydroxyl methyl cellulose, hydroxyethyl cellulose, hydroxypropyl cellulose hydroxypropylmethyl cellulose and combinations thereof, and an active component, wherein the ratio of glucan to water-soluble polymer by weight is 10:1 to 1:5, and wherein the viscosity of the film composition is 400 cps to 100,000 cps, said viscosity preventing components from settling, which would adversely affect the uniformity of the film.

2. The ingestible water-soluble delivery system of claim 1, wherein said glucan is selected from the group consisting of pullulan, elsinan and combinations thereof.

3. The ingestible water-soluble delivery system of claim 1, further comprising a water-soluble polymer selected from the group consisting of a gum, a protein, a starch, and combinations thereof.

4.   The ingestible water-soluble delivery system of claim 1, further comprising a water-soluble polymer selected from the group consisting of gum arabic, xanthan gum, tragacanth, acacia, carageenan, guar gum, locust bean gum, pectin, alginates and combinations thereof.

5.   The ingestible water-soluble delivery system of claim 1, further comprising a water-soluble polymer selected from the group consisting of polyvinyl alcohol, polyacrylic acid, polyvinyl pyrrolidone, poly(meth)acrylate, poly(meth) copolymers and combinations thereof.

6.   The ingestible water-soluble delivery system of claim 1, further comprising a starch selected from the group consisting of tapioca, rice, corn, potato, wheat and combinations thereof.

7.   The ingestible water-soluble delivery system of claim 6, wherein said starch is gelatinized, modified or unmodified.

8.   The ingestible water-soluble delivery system of claim 1, further comprising a protein selected from the group consisting of gelatin, zein, gluten, soy protein, soy protein isolate, whey protein, whey protein isolate, casein, levin, collagen and combinations thereof.

9.   The ingestible water-soluble delivery system of claim 1, further comprising a water-soluble polymer selected from the group consisting of dextrin, dextran and combinations thereof.

10.   The ingestible water-soluble delivery system of claim 1, further comprising a water-soluble polymer selected from the group consisting of chitin, chitosin or combinations thereof.

11.   The ingestible water-soluble delivery system of claim 1, further comprising polydextrose.

12.   The ingestible water-soluble delivery system of claim 1, wherein said active component is selected from the group consisting of cosmetic agents, pharmaceutical agents, bioactive agents and combinations thereof.

13.   The ingestible water-soluble delivery system of claim 1, wherein said active component is present in amounts of up to 60% by weight of the total composition.

14.   The ingestible water-soluble delivery system of claim 1, wherein said active component is present in amounts of up to 0.1 % to 60% by weight of the total composition.

15.   The ingestible water-soluble delivery system of claim 1, further comprising one or more members selected from the group consisting of taste-masking agents, plasticizing agents, surfactants, emulsifying agents, thickening agents, binding agents, cooling agents, saliva-stimulating agents, sweetening agents, antimicrobial agents and combinations thereof.

16.   A dried film formed from the composition of claim 1.

17.   The dried film of claim 16, formed from the composition of claim 1, wherein said active component is selected from the group consisting of cosmetic agents, pharmaceutical agents, bioactive agents and combinations thereof.

18.   The dried film formed from the composition of claims 16 or 17 having a thickness up to 5mm.

19.   The dried film of claims 16 or 17 having a polymeric carrier backing.

20.   The dried film of claims 16 or 17 in unit dosage form sealed in a pouch.

21.   A pharmaceutical and/or cosmetic dosage form comprising an ingestible water-soluble film composition comprising a glucan and a water-soluble polymer, comprising a cellulosic material, wherein said cellulosic material is selected from the group consisting of carboxymethyl cellulose, hydroxyl methyl cellulose, hydroxyethyl cellulose, hydroxypropyl cellulose hydroxypropylmethyl cellulose and combinations thereof, wherein the ratio of glucan to water-soluble polymer by weight is 10:1 to 1:5, and wherein the viscosity of the film composition is 400 cps to 100,000 cps, said viscosity preventing components from settling, which would adversely affect the uniformity of the film, said film being formed by depositing a wet film of said composition onto a substrate surface and controllably drying the wet film from the side contacting the substrate to prevent self-aggregation and achieve compositional uniformity to provide a film having no more than a 10% variance of a pharmaceutical and/or cosmetic active per unit area.

22. A pharmaceutical and/or cosmetic dosage form comprising a film having a uniformly dispersed composition comprising a glucan and a water-soluble polymer comprising a cellulosic material, wherein said cellulosic material is selected from the group consisting of carboxymethyl cellulose, hydroxyl methyl cellulose, hydroxyethyl cellulose, hydroxypropyl cellulose hydroxypropylmethyl cellulose and combinations thereof, a pharmaceutical and/or cosmetic active and a solvent, wherein the ratio of glucan to water-soluble polymer by weight is 10:1 to 1:5, and wherein the viscosity of the film composition is 400 cps to 100,000 cps, said viscosity preventing components from settling, which would adversely affect the uniformity of the film, said film being formed by depositing a wet film of said composition onto a substrate surface and controllably drying the wet film from the side contacting the substrate to prevent self-aggregation and achieve compositional uniformity.

23. A pharmaceutical composition in the form of a film for enteral or topical administration, comprising a composition having a uniformly distributed combination of a glucan, a water-soluble polymer comprising a cellulosic material, wherein said cellulosic material is selected from the group consisting of carboxymethyl cellulose, hydroxyl methyl cellulose, hydroxyethyl cellulose, hydroxypropyl cellulose hydroxypropylmethyl cellulose and combinations thereof, a polar solvent, and a pharmaceutical active, wherein the ratio of glucan to water-soluble polymer by weight is 10:1 to 1:5, and wherein the viscosity of the film composition is 400 cps to 100,000 cps, said viscosity preventing components from settling, which would adversely affect the uniformity of the film, said composition in its dried film form maintaining the uniform distribution of components through the application of controlled bottom drying of the film.

24. The ingestible water-soluble delivery system of claim 12, wherein said pharmaceutical active is selected from the group consisting of antimicrobial agents, non-steroidal antiinflammatory drugs, anti-tussives, decongestants, anti-histamines, expectorants, anti-diarrheals, $H_2$ antagonists, proton pump inhibitors, general non-selective CNS depressants, general non-selective CNS stimulants, selective CNS functional modifiers, anti-parkinsonism drugs, narcotics, analgesics, anti-pyretics, psychopharmacological drugs and combinations thereof.

25. The ingestible water-soluble delivery system of claim 1, wherein the film composition is essentially free of a surfactant.

26. The ingestible water-soluble delivery system of claims 1 or 25, wherein the film composition is essentially free of a plasticizer.

27. The ingestible water-soluble delivery system of claims 1, 25 or 26, wherein the film composition is essentially free of a polyalcohol.

28. The pharmaceutical and/or cosmetic dosage form of claims 21 or 22, wherein the film is essentially free of a surfactant.

29. The pharmaceutical and/or cosmetic dosage form of claims 21, 22, or 28, wherein the film is essentially free of a plasticizer.

30. The pharmaceutical and/or cosmetic dosage form of claims 21, 22, 28 or 29, wherein the film is essentially free of a polyalcohol.

31. The pharmaceutical composition of claim 23, wherein the film is essentially free of a surfactant.

32. The pharmaceutical composition of claims 23 or 31, wherein the film is essentially free of a plasticizer.

33. The pharmaceutical composition of claims 23, 31 or 32, wherein the film is essentially free of a polyalcohol.

34. The ingestible water-soluble delivery system of claim 1, wherein said cellulosic material comprises hydroxypropylmethyl cellulose.

**Patentansprüche**

1. Einnehmbares wasserlösliches Verabreichungssystem in Form einer Filmzusammensetzung umfassend ein Glucan, ein wasserlösliches Polymer umfassend ein Cellulosematerial, wobei das Cellulosematerial ausgewählt ist aus der Gruppe bestehend aus Carboxymethylcellulose, Hydroxylmethylcellulose, Hydroxyethylcellulose, Hydroxypropylcellulose, Hydroxypropylmethylcellulose und Kombinationen davon, und eine Wirkungskomponente, wobei das Verhältnis von Glucan zu wasserlöslichem Polymer, bezogen auf das Gewicht, 10:1 bis 1:5 beträgt, und wobei die Viskosität der Filmzusammensetzung 400 cps bis 100000 cps beträgt, wobei diese Viskosität verhindert, dass sich Komponenten absetzen, was die Einheitlichkeit des Films beeinträchtigen würde.

2. Einnehmbares wasserlösliches Verabreichungssystem nach Anspruch 1, wobei das Glucan ausgewählt ist aus der Gruppe bestehend aus Pullulan, Elsinan und Kombinationen davon.

3. Einnehmbares wasserlösliches Verabreichungssystem nach Anspruch 1, außerdem umfassend ein wasserlösliches Polymer, ausgewählt aus der Gruppe bestehend aus einem Gummi, einem Protein, einer Stärke und Kombinationen davon.

4. Einnehmbares wasserlösliches Verabreichungssystem nach Anspruch 1, außerdem umfassend ein wasserlösliches Polymer, ausgewählt aus der Gruppe bestehend aus Gummi arabicum, Xanthangummi, Tragant, Akaziengummi, Carrageen, Guaran, Johannisbrotgummi, Pektin, Alginaten und Kombinationen davon.

5. Einnehmbares wasserlösliches Verabreichungssystem nach Anspruch 1, außerdem umfassend ein wasserlösliches Polymer, ausgewählt aus der Gruppe bestehend aus Polyvinylalkohol, Polyacrylsäure, Polyvinylpyrrolidon, Poly (meth)acrylat, Poly(meth)copolmeren und Kombinationen davon.

6. Einnehmbares wasserlösliches Verabreichungssystem nach Anspruch 1, außerdem umfassend eine Stärke, ausgewählt aus der Gruppe bestehend aus Tapioka, Reis, Mais, Kartoffel, Weizen und Kombinationen davon.

7. Einnehmbares wasserlösliches Verabreichungssystem nach Anspruch 6, wobei die Stärke gelatinisiert, modifiziert oder unmodifiziert ist.

8. Einnehmbares wasserlösliches Verabreichungssystem nach Anspruch 1, außerdem umfassend ein Protein, ausgewählt aus der Gruppe bestehend aus Gelatine, Zein, Gluten, Sojaprotein, Sojaproteinisolat, Molkenprotein, Molkenproteinisolat, Casein, Levin, Collagen und Kombinationen davon.

9. Einnehmbares wasserlösliches Verabreichungssystem nach Anspruch 1, außerdem umfassend ein wasserlösliches Polymer, ausgewählt aus der Gruppe bestehend aus Dextrin, Dextran und Kombinationen davon.

10. Einnehmbares wasserlösliches Verabreichungssystem nach Anspruch 1, außerdem umfassend ein wasserlösliches Polymer, ausgewählt aus der Gruppe bestehend aus Chitin, Chitosin oder Kombinationen davon.

11. Einnehmbares wasserlösliches Verabreichungssystem nach Anspruch 1, außerdem umfassend Polydextrose.

12. Einnehmbares wasserlösliches Verabreichungssystem nach Anspruch 1, wobei die Wirkungskomponente ausgewählt ist aus der Gruppe bestehend aus kosmetischen Wirkstoffen, pharmazeutischen Wirkstoffen, bioaktiven Wirkstoffen und Kombinationen davon.

13. Einnehmbares wasserlösliches Verabreichungssystem nach Anspruch 1, wobei die Wirkungskomponente in Mengen von bis zu 60 Gew.-% der Gesamtzusammensetzung vorhanden ist.

14. Einnehmbares wasserlösliches Verabreichungssystem nach Anspruch 1, wobei die Wirkungskomponente in Mengen von bis zu 0,1 bis 60 Gew.-% der Gesamtzusammensetzung vorhanden ist.

15. Einnehmbares wasserlösliches Verabreichungssystem nach Anspruch 1, außerdem umfassend einen oder mehrere Bestandteile, ausgewählt aus der Gruppe bestehend aus Geschmacksmaskierungsmitteln, Weichmachern, oberflächenaktiven Substanzen, Emulgiermitteln, Verdickungsmitteln, Bindemitteln, kühlenden Mitteln, speichelstimulierenden Mitteln, Süßungsmitteln, antimikrobiellen Mitteln und Kombinationen davon.

16. Getrockneter Film, gebildet aus der Zusammensetzung nach Anspruch 1.

17. Getrockneter Film nach Anspruch 16, gebildet aus der Zusammensetzung nach Anspruch 1, wobei die Wirkungskomponente ausgewählt ist aus der Gruppe bestehend aus kosmetischen Wirkstoffen, pharmazeutischen Wirkstoffen, bioaktiven Wirkstoffen und Kombinationen davon.

**18.** Getrockneter Film, gebildet aus der Zusammensetzung nach den Ansprüchen 16 oder 17 mit einer Dicke bis zu 5 mm.

**19.** Getrockneter Film nach den Ansprüchen 16 oder 17 mit einer polymeren Trägerschicht.

**20.** Getrockneter Film nach den Ansprüchen 16 oder 17 in Dosierungseinheitsform, eingeschlossen in einem Beutel.

**21.** Pharmazeutische und/oder kosmetische Dosierungsform, umfassend eine einnehmbare wasserlösliche Filmzusammensetzung umfassend ein Glucan und ein wasserlösliches Polymer, umfassend ein Cellulosematerial, wobei das Cellulosematerial ausgewählt ist aus der Gruppe bestehend aus Carboxymethylcellulose, Hydroxylmethylcellulose, Hydroxyethylcellulose, Hydroxypropylcellulose, Hydroxypropylmethylcellulose und Kombinationen davon, wobei das Verhältnis von Glucan zu wasserlöslichem Polymer, bezogen auf das Gewicht, 10:1 bis 1:5 beträgt, und wobei die Viskosität der Filmzusammensetzung 400 cps bis 100000 cps beträgt, wobei diese Viskosität verhindert, dass sich Komponenten absetzen, was die Einheitlichkeit des Films beeinträchtigen würde, wobei der Film gebildet wird durch Abscheiden eines Nassfilms der Zusammensetzung auf eine Substratoberfläche und kontrollierbares Trocknen des Nassfilms von der Seite her, die das Substrat berührt, um eine Selbstaggregation zu verhindern und eine Einheitlichkeit der Zusammensetzung zu erzielen, um einen Film mit nicht mehr als einer 10 %igen Varianz eines pharmazeutischen und/oder kosmetischen Wirkstoffs pro Flächeneinheit bereitzustellen.

**22.** Pharmazeutische und/oder kosmetische Dosierungsform, umfassend einen Film mit einer einheitlich dispergierten Zusammensetzung umfassend ein Glucan und ein wasserlösliches Polymer umfassend ein Cellulosematerial, wobei das Cellulosematerial ausgewählt ist aus der Gruppe bestehend aus Carboxymethylcellulose, Hydroxylmethylcellulose, Hydroxyethylcellulose, Hydroxypropylcellulose, Hydroxypropylmethylcellulose und Kombinationen davon, einen pharmazeutischen und/oder kosmetischen Wirkstoff und ein Lösungsmittel, wobei das Verhältnis von Glucan zu wasserlöslichem Polymer, bezogen auf das Gewicht, 10:1 bis 1:5 beträgt, und wobei die Viskosität der Filmzusammensetzung 400 cps bis 100000 cps beträgt, wobei diese Viskosität verhindert, dass sich Komponenten absetzen, was die Einheitlichkeit des Films beeinträchtigen würde, wobei der Film gebildet wird durch Abscheiden eines Nassfilms der Zusammensetzung auf eine Substratoberfläche und kontrollierbares Trocknen des Nassfilms von der Seite her, die das Substrat berührt, um eine Selbstaggregation zu verhindern und eine Einheitlichkeit der Zusammensetzung zu erzielen.

**23.** Pharmazeutische Zusammensetzung in Form eines Films für eine enterale oder topische Verabreichung, umfassend eine Zusammensetzung mit einer einheitlich verteilten Kombination von einem Glucan, einem wasserlöslichen Polymer umfassend ein Cellulosematerial, wobei das Cellulosematerial ausgewählt ist aus der Gruppe bestehend aus Carboxymethylcellulose, Hydroxylmethylcellulose, Hydroxyethylcellulose, Hydroxypropylcellulose, Hydroxypropylmethylcellulose und Kombinationen davon, einem polaren Lösungsmittel und einem pharmazeutischen Wirkstoff, wobei das Verhältnis von Glucan zu wasserlöslichem Polymer, bezogen auf das Gewicht, 10:1 bis 1:5 beträgt, und wobei die Viskosität der Filmzusammensetzung 400 cps bis 100000 cps beträgt, wobei diese Viskosität verhindert, dass sich Komponenten absetzen, was die Einheitlichkeit des Films beeinträchtigen würde, wobei die Zusammensetzung in ihrer getrockneten Filmform die einheitliche Verteilung von Komponenten durch die Anwendung einer kontrollierten Trocknung des Films von unten aufrechterhält.

**24.** Einnehmbares wasserlösliches Verabreichungssystem nach Anspruch 12, wobei der pharmazeutische Wirkstoff ausgewählt ist aus der Gruppe bestehend aus antimikrobiellen Wirkstoffen, nicht-steroidalen Entzündungshemmern, Hustenmitteln, abschwellenden Mitteln, Antihistaminen, auswurffördernden Mitteln, Antidiarrhoika, $H_2$-Antagonisten, Protonenpumpenhemmern, allgemeinen nicht-selektiven ZNS-Sedativa, allgemeinen nicht-selektiven ZNS-Stimulanzien, selektiven ZNS-Funktionsmodifikatoren, Anti-Parkinson-Mitteln, Narkotika, Analgetika, Antipyretika, Psychopharmaka und Kombinationen davon.

**25.** Einnehmbares wasserlösliches Verabreichungssystem nach Anspruch 1, wobei die Filmzusammensetzung im Wesentlichen frei von einer oberflächenaktiven Substanz ist.

**26.** Einnehmbares wasserlösliches Verabreichungssystem nach den Ansprüchen 1 oder 25, wobei die Filmzusammensetzung im Wesentlichen frei von einem Weichmacher ist.

**27.** Einnehmbares wasserlösliches Verabreichungssystem nach den Ansprüchen 1, 25 oder 26, wobei die Filmzusammensetzung im Wesentlichen frei von einem Polyalkohol ist.

**28.** Pharmazeutische und/oder kosmetische Dosierungsform nach den Ansprüchen 21 oder 22, wobei der Film im Wesentlichen frei von einer oberflächenaktiven Substanz ist.

29. Pharmazeutische und/oder kosmetische Dosierungsform nach den Ansprüchen 21, 22 oder 28, wobei der Film im Wesentlichen frei von einem Weichmacher ist.

30. Pharmazeutische und/oder kosmetische Dosierungsform nach den Ansprüchen 21, 22, 28 oder 29, wobei der Film im Wesentlichen frei von einem Polyalkohol ist.

31. Pharmazeutische Zusammensetzung nach Anspruch 23, wobei der Film im Wesentlichen frei von einer oberflächenaktiven Substanz ist.

32. Pharmazeutische Zusammensetzung nach den Ansprüchen 23 oder 31, wobei der Film im Wesentlichen frei von einem Weichmacher ist.

33. Pharmazeutische Zusammensetzung nach den Ansprüchen 23, 31 oder 32, wobei der Film im Wesentlichen frei von einem Polyalkohol ist.

34. Einnehmbares wasserlösliches Verabreichungssystem nach Anspruch 1, wobei das Cellulosematerial Hydroxypropylmethylcellulose umfasst.


**Revendications**

1. Système de délivrance hydrosoluble ingérable sous la forme d'une composition de film qui comprend un glucane, un polymère hydrosoluble comprenant une substance cellulosique, ladite substance cellulosique étant choisie dans le groupe constitué par la carboxyméthyl cellulose, l'hydroxyl méthyl cellulose, l'hydroxyéthyl cellulose, l'hydroxypropyl cellulose, l'hydroxypropyl méthyl cellulose et leurs combinaisons, et un composant actif, le rapport en poids du glucane sur le polymère hydrosoluble étant de 10/1 à 1/5, et la viscosité de la composition de film étant de 400 cps à 100 000 cps, ladite viscosité empêchant la sédimentation des composants, laquelle affecterait négativement l'uniformité du film.

2. Système de délivrance hydrosoluble ingérable selon la revendication 1, ledit glucane étant choisi dans le groupe constitué par le pullulane, l'elsinane et leurs combinaisons.

3. Système de délivrance hydrosoluble ingérable selon la revendication 1, comprenant en outre un polymère hydrosoluble choisi dans le groupe constitué par une gomme, une protéine, un amidon, et leurs combinaisons.

4. Système de délivrance hydrosoluble ingérable selon la revendication 1, comprenant en outre un polymère hydrosoluble choisi dans le groupe constitué par la gomme arabique, la gomme de xanthane, la tragacanthe, l'acacia, la carraghénane, la gomme de guar, la gomme de caroubier, la pectine, les alginates et leurs combinaisons.

5. Système de délivrance hydrosoluble ingérable selon la revendication 1, comprenant en outre un polymère hydrosoluble choisi dans le groupe constitué par l'alcool polyvinylique, l'acide polyacrylique, la polyvinylpyrrolidone, le poly(méth)acrylate, les poly(méth)copolymères et leurs combinaisons.

6. Système de délivrance hydrosoluble ingérable selon la revendication 1, comprenant en outre un amidon choisi dans le groupe constitué par le tapioca, le riz, le maïs, la pomme de terre, le blé et leurs combinaisons.

7. Système de délivrance hydrosoluble ingérable selon la revendication 6, ledit amidon étant gélatinisé, modifié ou non modifié.

8. Système de délivrance hydrosoluble ingérable selon la revendication 1, comprenant en outre une protéine choisie dans le groupe constitué par la gélatine, la zéine, le gluten, la protéine de soja, un isolat de protéine de soja, la protéine du lactosérum, un isolat de protéine du lactosérum, la caséine, la lévine, le collagène et leurs combinaisons.

9. Système de délivrance hydrosoluble ingérable selon la revendication 1, comprenant en outre un polymère hydrosoluble choisi dans le groupe constitué par la dextrine, le dextrane et leurs combinaisons.

**10.** Système de délivrance hydrosoluble ingérable selon la revendication 1, comprenant en outre un polymère hydrosoluble choisi dans le groupe constitué par la chitine, la chitosine ou leurs combinaisons.

**11.** Système de délivrance hydrosoluble ingérable selon la revendication 1, comprenant en outre du polydextrose.

**12.** Système de délivrance hydrosoluble ingérable selon la revendication 1, ledit composant actif étant choisi dans le groupe constitué par les agents cosmétiques, les agents pharmaceutiques, les agents bioactifs et leurs combinaisons.

**13.** Système de délivrance hydrosoluble ingérable selon la revendication 1, ledit composant actif étant présent en des quantités pouvant atteindre 60 % en poids de la composition totale.

**14.** Système de délivrance hydrosoluble ingérable selon la revendication 1, ledit composant actif étant présent en quantités pouvant atteindre 0,1 % à 60 % en poids de la composition totale.

**15.** Système de délivrance hydrosoluble ingérable selon la revendication 1, comprenant en outre un ou plusieurs éléments choisis dans le groupe constitué par les agents de masquage du goût, les agents plastifiants, les surfactants, les agents émulsifiants, les agents épaississants, les agents de liaison, les agents de refroidissement, les agents stimulant la salive, les agents édulcorants, les agents antimicrobiens et leurs combinaisons.

**16.** Film sec formé à partir de la composition selon la revendication 1.

**17.** Film sec selon la revendication 16, formé à partir de la composition selon la revendication 1, ledit composant actif étant choisi dans le groupe constitué par les agents cosmétiques, les agents pharmaceutiques, les agents bioactifs et leurs combinaisons.

**18.** Film sec formé à partir de la composition selon les revendications 16 ou 17, ayant une épaisseur pouvant atteindre jusqu'à 5 mm.

**19.** Film sec selon les revendications 16 ou 17, ayant un renfort support polymère.

**20.** Film sec selon les revendications 16 ou 17, en une forme posologique unitaire enfermée dans une poche.

**21.** Forme posologique pharmaceutique et/ou cosmétique comprenant une composition de film hydrosoluble ingérable, comprenant un glucane et un polymère hydrosoluble, comprenant une substance cellulosique, ladite substance cellulosique étant choisie dans le groupe constitué par la carboxyméthyl cellulose, l'hydroxyl méthyl cellulose, l'hydroxyéthyl cellulose, l'hydroxypropyl cellulose, l'hydroxypropyl méthyl cellulose et leurs combinaisons, le rapport en poids du glucane sur le polymère hydrosoluble étant de 10/1 à 1/5, et la viscosité de la composition de film étant de 400 cps à 100 000 cps, ladite viscosité empêchant la sédimentation des composants, ce qui affecterait négativement l'uniformité du film, ledit film étant formé par dépôt d'un film mouillé de ladite composition sur une surface de substrat et séchage contrôlé du film mouillé du côté en contact avec le substrat pour empêcher l'auto-agrégation et obtenir une uniformité de composition pour fournir un film dont la variance n'est pas supérieure à 10 % d'un principe actif pharmaceutique et/ou cosmétique par aire unitaire.

**22.** Forme posologique pharmaceutique et/ou cosmétique comprenant un film ayant une composition dispersée uniformément comprenant un glucane et un polymère hydrosoluble comprenant une substance cellulosique, ladite substance cellulosique étant choisie dans le groupe constitué par la carboxyméthyl cellulose, l'hydroxyl méthyl cellulose, l'hydroxyéthyl cellulose, l'hydroxypropyl cellulose, l'hydroxypropyl méthyl cellulose et leurs combinaisons, un principe actif pharmaceutique et/ou cosmétique et un solvant, le rapport en poids du glucane sur le polymère hydrosoluble étant de 10/1 à 1/5, et la viscosité de la composition de film étant de 400 cps à 100 000 cps, ladite viscosité empêchant la sédimentation des composants, laquelle affecterait négativement l'uniformité du film, ledit film étant formé par dépôt d'un film mouillé de ladite composition sur une surface de substrat et par séchage contrôlé du film mouillé d'un côté en contact avec le substrat pour empêcher l'auto-agrégation et obtenir une uniformité de la composition.

**23.** Composition pharmaceutique sous la forme d'un film pour une administration entérale ou topique, qui comprend une composition ayant une combinaison uniformément distribuée d'un glucane, d'un polymère hydrosoluble comprenant une substance cellulosique, ladite substance cellulosique étant choisie dans le groupe constitué par la carboxyméthyl cellulose, l'hydroxyl méthyl cellulose, l'hydroxyéthyl cellulose, l'hydroxypropyl cellulose, l'hydroxypropyl méthyl cellulose et leurs combinaisons, d'un solvant polaire, et d'un principe actif pharmaceutique, le rapport en poids du glucane sur le polymère hydrosoluble étant de 10/1 à 1/5, et la viscosité de la composition de film étant de 400 cps à 100 000 cps, ladite viscosité empêchant la sédimentation des composants, ce qui affecterait négativement l'uniformité du film, ladite composition sous sa forme de film sec maintenant la distribution uniforme de composés par l'application du séchage à coeur contrôlé du film.

24. Système de délivrance hydrosoluble ingérable selon la revendication 12, ledit principe actif pharmaceutique étant choisi dans le groupe constitué par les agents antimicrobiens, les anti-inflammatoires non stéroïdiens, les antitussifs, les décongestionnants, les antihistaminiques, les expectorants, les antidiarrhéiques, les antagonistes d'H2, les inhibiteurs de la pompe à protons, les dépresseurs non sélectifs et généraux du SNC, les stimulants non sélectifs et généraux du SNC, les modificateurs fonctionnels et sélectifs du SNC, les antiparkinsoniens, les narcotiques, les analgésiques, les antipyrétiques, les médicaments psychopharmacologiques et leurs combinaisons.

25. Système de délivrance hydrosoluble ingérable selon la revendication 1, la composition de film étant sensiblement dépourvue de surfactant.

26. Système de délivrance hydrosoluble ingérable selon les revendications 1 ou 25, la composition de film étant sensiblement dépourvue de plastifiant.

27. Système de délivrance hydrosoluble ingérable selon les revendications 1, 25 ou 26, la composition de film étant sensiblement dépourvue de polyalcool.

28. Forme posologique pharmaceutique et/ou cosmétique selon les revendications 21 ou 22, le film étant sensiblement dépourvu de surfactant.

29. Forme posologique pharmaceutique et/ou cosmétique selon les revendications 21, 22 ou 28, le film étant sensiblement dépourvu de plastifiant.

30. Forme posologique pharmaceutique et/ou cosmétique selon les revendications 21, 22, 28 ou 29, le film étant sensiblement dépourvu de polyalcool.

31. Composition pharmaceutique selon la revendication 23, le film étant sensiblement dépourvu de surfactant.

32. Composition pharmaceutique selon les revendications 23 ou 31, le film étant sensiblement dépourvu de plastifiant.

33. Composition pharmaceutique selon les revendications 23, 31 ou 32, le film étant sensiblement dépourvu de polyalcool.

34. Système de délivrance hydrosoluble et ingérable selon la revendication 1, ladite substance cellulosique comprenant l'hydroxypropylméthyl cellulose.

**FIG 1**

**FIG 2**

**FIG 3**

**FIG 4**

**FIG 5**

**FIG. 6**

# FIG. 7

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 4562020 A, Haijiya
- US 4927636 A, Haijiya
- US 5411945 A, Ozaki
- US 5518902 A, Ozaki
- US 20010022964 A1, Leung
- WO 0170194 A
- US 074272 A
- US 4631837 A